(19) Europäisches Patentamt / European Patent Office / Office européen des brevets

(11) **EP 2 696 911 B1**

(12) **EUROPÄISCHE PATENTSCHRIFT**

(45) Veröffentlichungstag und Bekanntmachung des Hinweises auf die Patenterteilung:
**18.05.2016 Patentblatt 2016/20**

(21) Anmeldenummer: **12717250.0**

(22) Anmeldetag: **16.04.2012**

(51) Int Cl.:
*A61M 1/16* (2006.01)  *A61M 1/34* (2006.01)

(86) Internationale Anmeldenummer:
**PCT/EP2012/056951**

(87) Internationale Veröffentlichungsnummer:
**WO 2012/140275 (18.10.2012 Gazette 2012/42)**

(54) **VERFAHREN UND VORRICHTUNG ZUR ANPASSUNG DER MITTELMOLEKULAREN REINIGUNGSLEISTUNG DURCH EINSTELLUNG DES SUBSTITUTIONSFLUSSES**

METHOD AND DEVICE FOR ADAPTING THE MEAN MOLECULAR CLEANING PERFORMANCE BY SETTING THE SUBSTITUTION FLOW

PROCÉDÉ ET DISPOSITIF DESTINÉS À ADAPTER LA PERFORMANCE D'ÉPURATION DANS LE DOMAINE DES TAILLES MOLÉCULAIRES INTERMÉDIAIRES EN AJUSTANT LE FLUX DE SUBSTITUTION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.04.2011 DE 102011018262**

(43) Veröffentlichungstag der Anmeldung:
**19.02.2014 Patentblatt 2014/08**

(73) Patentinhaber: **B. Braun Avitum AG**
**34212 Melsungen (DE)**

(72) Erfinder:
• **MOLL, Stefan**
**22587 Hamburg (DE)**

• **WOLFF, Henrik**
**37213 Witzenhausen (DE)**

(74) Vertreter: **Winter, Brandl, Fürniss, Hübner, Röss, Kaiser, Polte - Partnerschaft mbB Patent- und Rechtsanwaltskanzlei Bavariaring 10 80336 München (DE)**

(56) Entgegenhaltungen:
**EP-A1- 1 175 917        US-A- 5 507 723**
**US-A1- 2005 251 086     US-A1- 2008 215 247**
**US-B1- 6 666 840**

## Beschreibung

Die vorliegende Erfindung bezieht sich auf ein Verfahren zur Bestimmung einer Flussrate einer Substitutionslösung, bei der eine Reinigungsleistung für mittelmolekulare Substanzen eines Tangentialflussfilters einer Blutbehandlungseinheit erreicht wird, die durch einen vorher festgelegten Bereich definiert ist.

Die Erfindung bezieht sich dabei auf das Feld der Filtration, genauer gesagt der Tangentialflussfiltration (TFF). Hierbei ist die zu reinigende Flüssigkeit durch eine semipermeable Membran von der Reinigungslösung getrennt. Typischerweise weist die Reinigungslösung eine niedrigere Konzentration derjenigen Stoffe als in der zu reinigenden Flüssigkeit auf, aus der sie entfernt werden sollen. Durch diesen Konzentrationsgradienten wird über eine Austauschoberfläche Diffusion erzeugt. Um die Diffusion als reinigende Kraft optimal nutzen zu können, werden Tangentialflussfilter typischerweise im Gegenstromprinzip betrieben.

Ein Dialysefilter (Dialysator) besteht im Wesentlichen aus Hohlfasern, also zylinderförmigen Fasern, die in Längsausrichtung ein Gehäuse durchziehen. Die Wände der Hohlfasern weisen teildurchlässige (semipermeable) Strukturen auf und wirken als Membranen. An ihren Enden sind die Hohlfasern in eine Vergussmasse eingebettet. Im Dialysefilter können die Hohlfasern zu Modulen mit mehreren Quadratmetern Filterfläche zusammengefasst werden. Bei der Dialyse durch Tangentialflussfiltration, auch Querstromfiltration oder Cross-Flow-Filtration genannt, wird den Hohlfasern über einen ersten Flüssigkeitskreislauf Blut/Plasma zugeführt, das sie der Länge nach durchströmt. Durch einen zweiten Flüssigkeitskreislauf wird die Dialyseflüssigkeit, in der Regel im Gegenstromprinzip, aber ggf. auch parallel zum Blutstrom, zugeführt. Das Gehäuse weist daher vier Anschlüsse auf, jeweils eine Zufuhr und eine Abfuhr für jeden der beiden Flüssigkeitsströme. An der Innenseite der Hohlfasermembran verläuft der Blutstrom und auf der Außenseite strömt die Dialyseflüssigkeit. Dem Reinigungseffekt liegt eine Diffusion der betreffenden Stoffe gemäß dem Konzentrationsgradienten des Blutes und der Dialyselösung zu Grunde.

Ein weiterer Reinigungsmechanismus ist die Konvektion. Hierbei wird ein Druckgradient über die semipermeable Membran erzeugt, wodurch die zu reinigende Flüssigkeit über die semipermeable Membran gedrückt wird. Dadurch werden die zu entfernenden Substanzen in der Konzentration, in der sie vorliegen, mitgespült. Dieser Reinigungsprozess ist daher nicht von der Konzentration der zu entfernenden Substanzen in der Reinigungslösung abhängig, entscheidend sind hier nur deren Konzentration in der zu reinigenden Flüssigkeit und die Membraneigenschaften wie beispielsweise Siebkoeffizient und Permeabilität und natürlich der Ultrafiltrationsfluss bzw. der Substitutionsfluss. Es ist daher von Interesse, diesen auf einen optimalen Wert einzustellen.

Bei Patienten mit teilweiser oder vollständiger Niereninsuffizienz werden Abfallprodukte des natürlichen Stoffwechsels einschließlich urämischer Toxine durch Blutbehandlungsverfahren, wie beispielsweise der Hämodialyse oder die Hämodiafiltration entfernt, wobei die Entfernung der Stoffe aus dem Blut extrakorporal durch den Kontakt des Blutes mit einer Dialyselösung erfolgt. Der Stofftransport vom Blut in die Dialyselösung erfolgt über diffusive und konvektive Effekte. Ziel ist, dass vornehmlich urämische Toxine aus dem Blut entfernt werden. Dies geschieht, indem für den Patienten lebensnotwendige Substanzen in physiologischer Konzentration der Dialyselösung beigefügt werden. Somit existiert für diese Substanzen kein Konzentrationsgefälle über die Dialysatormembran und sie werden dem Blut nicht entzogen.

Das Maß für die Dialysedosis eines Patienten kann nicht nur auf Basis der zumeist subjektiven Einschätzung der Gesundheit des Patienten erfolgen. Es ist notwendig, den Dialyseerfolg verlässlich zu quantifizieren, damit eine ausreichende Dialyseleistung sichergestellt wird. Gleichzeitig ist ein zu hohes Maß an Dialyse aus Kostengründen ebenfalls zu vermeiden. Um die Dialysetherapie effizienter zu gestalten, ist es notwendig, die Dialyseeffizienz während der Behandlung zu kontrollieren, um diese durch Anpassung der variablen Parameter der Blutbehandlungseinheit manuell oder automatisch steuern zu können.

Um eine adäquate Dialysetherapie zu gewährleisten, wurde das $Kt/V_{urea}$-Modell entwickelt. Harnstoff (Urea) stellt das wichtigste Stoffwechselendprodukt im zu reinigenden Blut dar. Daher wird Harnstoff zur Bestimmung einer adäquaten Dialysetherapie herangezogen. In Analogie zu Harnstoff als kleinmolekularem Marker kann beispielsweise auch der $Kt/V_{beta2-Mikroglobulin}$ mit beta2-Mikroglobulin als typischem Mittelmolekül verwendet werden, um die Qualität der mittelmolekularen Reinigung zu quantifizieren. Anstelle von beta2-Mikroglobulin können natürlich auch andere Mittelmoleküle, d.h. mittelmolekulare Moleküle als Marker dienen.

$K$ ist die Reinigungsleistung (Clearance) des Dialysators bezogen auf den jeweiligen Stoff aus dem Blut in ml/min, t die Behandlungszeit in min und V das Verteilungsvolumen von Harnstoff in ml im menschlichen Körper, das in direkter Relation zum Gewicht des Patienten steht. In der Regel sind dies 60% der Körpermasse bzw. des Körpergewichts, in der das Blut zirkulieren kann (Körperwassergehalt). Der dimensionslose Faktor $Kt/V$ bildet ein Maß für die Reduktion eines bestimmten Stoffes oder einer Stoffklasse aus dem Blut. Ziel einer Behandlung ist es im Fall des Harnstoffs, einen Kt/V $\geq$ 1,2 zu erzielen. Bei einem normalen Behandlungsverlauf werden Werte erreicht, die dieses Kriterium in der Regel erfüllen. Jedoch können bei einer Behandlung Widrigkeiten auftreten, die sich negativ auf den Behandlungsverlauf sowie das Behandlungsergebnis auswirken. Es ist somit wichtig, während einer Behandlung die Einflussparameter zu überwachen und zu kontrollieren, um auf solche Widrigkeiten schnell und vor allem gezielt reagieren und die geeigneten Systemparameter während der Dialyse entsprechend anpassen zu können.

Im Gegensatz zur Quantifizierung der kleinmolekularen Reinigung mit Harnstoff als Marker, gibt es für die Quantifizierung der mittelmolekularen Reinigung noch keinen eigenständigen allgemeingültigen Parameter. Die Quantifizierung orientiert sich daher an den für die kleinmolekulare Reinigung etablierten Modellen.

Bei modernen Dialysatoren wird eine Reinigung durch Ausnutzen der Konvektion durch das Prinzip der Ultrafiltration erreicht. Hierbei werden nicht nur die zu entfernenden oder harnpflichtigen Substanzen aus dem Blut entfernt. Aufgrund des über die Ultrafiltrationspumpe (UF-Pumpe) angelegten Druckgradienten (Transmembrandruck) an der Filtermembran werden dem Blut konvektiv Plasmaanteile über die Membran entzogen. Zu diesem Zweck wird auf der Dialysatseite aus einem geschlossenen System eine definierte Menge Dialyselösung entnommen. Dadurch wird in dem geschlossenen System ein Unterdruck erzeugt, der dafür sorgt, dass die gleiche Menge Blut über die semipermeable Membran zur Dialysatseite übertritt.

Der Volumenverlust an Plasmaanteilen muß durch Zuführung von Substitutionslösung ausgeglichen werden. Die Substitutionslösung ist typischerweise eine Elektrolytlösung. In diesem Zusammenhang ist zwischen drei verschiedenen Begriffen zu unterscheiden. Der Weightloss beschreibt die Menge an Flüssigkeit, die dem Kreislauf effektiv entnommen wird. Das Substitutionsvolumen ist die Flüssigkeitsmenge, die dem Patienten als Ausgleich für den Volumenverlust, der über den Weightloss hinausgeht, zurückgegeben wird und das Ultrafiltrationsvolumen ist die Flüssigkeitsmenge, die insgesamt von der Blut- auf die Dialysatseite übertritt, also die Summe aus Weightloss und Substitution.

Eine andere Betrachtungsweise kennt nur die Ultrafiltration, für den effektiven Flüssigkeitsverlust des Patienten und die Substitution als die Ausgleichsmenge für den zu starken Flüssigkeitsverlust einer Hämodiafiltrations- (HDF-) Therapie.

Neuere Untersuchungen gehen heute aber davon aus, dass insbesondere mittelgroße Moleküle für die Entwicklung der Urämie und den daraus resultierenden Langzeitkomplikationen der chronischen Niereninsuffizienz in einem wesentlichen Maße beitragen. Das Molekulargewicht mittelmolekularer Substanzen bewegt sich zwischen 500 und 50000 Dalton. Die wichtigsten mittelmolekularen Substanzen im Blut sind Vitamin $B_{12}$ (1355 Dalton), Inulin (5200 Dalton), Cystatin C (13360 Dalton), Retinol Binding Protein (RBP; 15000 Dalton), Complement Faktor D (23500 Dalton) und $\beta_2$-Mikroglobulin (11900 Dalton). Allerdings sind eine Vielzahl weiterer unbekannter mittelmolekularer Substanzen im Blut vorhanden, die Langzeitkomplikationen verursachen können. Eine Entfernung dieser mittelmolekularen Substanzen wurde in mehreren Studien mit einer erhöhten Lebenserwartung in Verbindung gebracht (Port et al, AJKD 2001, 37: 276-86; Locatelli et al, Kidney Int.1999; 55: 286-93).

Die Entfernung dieser mittelmolekularen Substanzen wird durch die heute gängigen high-flux Dialysatoren geleistet und erfolgt hauptsächlich über die Ultrafiltration, wie z.B. bei der Hämofiltration bzw. der Hämodiafiltration, wobei kontrolliert Flüssigkeit dem Blut des Patienten entzogen wird. Hierzu wird ein Druckgradient über die semipermeable Membran des Dialysators erzeugt. Dadurch wird Plasmaflüssigkeit dem Blut konvektiv entzogen (Ultrafiltration). Daher ist es von Interesse, den Substitutionsfluss auf einen optimalen Wert einzustellen, um eine optimierte Reinigungsleistung zu erzielen.

Ein entscheidender Prozess für die beschriebene Konvektion ist die Interaktion der Filtermembran mit dem Blut. Durch diese kontinuierliche Einwirkung verschlechtern sich die Flusseigenschaften des Filters sowohl in Transmembranrichtung als auch in Blutflussrichtung. Transmembranrichtung oder transmembranös bezieht sich hierbei auf einen Fluss des Blutes über die Membran des Dialysators bzw. Dialysefilters. Diese Veränderungen werden beispielsweise durch Thrombozytenanlagerung auf der Membran, Koagelbildung, chemisches Binden von Blutbestandteilen an die Membran oder schlicht mechanisches (strömungsbedingtes) Drücken der Blutbestandteile an und sogar in die Membran verursacht.

Bei der Koagelbildung entsteht ein gallertartiges durch Fibrinfäden stabilisiertes Aggregat aus roten Blutkörperchen (Erythrozyten). Anders als der Begriff Thrombus beschreibt ein Koagulum ein Blutgerinnsel, welches sich außerhalb eines Blut- oder Lymphgefäßes (extravasal) befindet und nicht innerhalb (intravasal).

Besonders die Entfernung von mittelmolekularen Substanzen durch Konvektion wird durch die genannten Faktoren beeinträchtigt, da wie gesagt die mittelmolekularen Substanzen im Gegensatz zu den niedermolekularen Substanzen nicht durch Diffusion, sondern vorwiegend nur durch Konvektion entfernt werden. Durch Verschlechterung der transmembranen Flusseigenschaften oder der Permeabilität, verschlechtert sich vor allem der Siebkoeffizient für die urämischen Substanzen im mittelmolekularen Bereich, was dazu führt, dass bei gleicher Menge konvektiv gefilterter Flüssigkeit weniger dieser urämischen Substanzen aus dem Blutkreislauf entfernt werden. Ein anderer Effekt ist die Verringerung der effektiven Durchflussfläche sowohl in Blutflussrichtung als auch in Transmembranrichtung. Dies hat eine Reduzierung der aktiven Filteroberfläche zur Folge, wodurch es zu einer Verschlechterung der diffusiven Reinigung um bis zu > 50% kommen kann. Bei neuwertigen Filtern besteht in der Regel ein Pufferpotential, das größer ist als die maximale physiologische Filterung. Dadurch kann eine Verringerung der effektiven Durchflussfläche in einem bestimmten Maß abgefangen werden. Ist dieses Potential jedoch ausgeschöpft, kommt es zu den zuvor beschriebenen Effekten.

Als geeignete Gegenmaßnahmen oder Reaktionen auf solche Änderungen gelten allgemein das Spülen mit Kochsalzlösung zum "Säubern" des Dialysators, die Zugabe von Heparin, um eine weitere Koagelbildung zu verhindern oder das Senken der Ultrafiltrationsrate (UF), um die Hämokonzentration zu reduzieren. Die Permeabilität der Membran wird bestimmt durch Messung des Flüssigkeitsvolumens, das bei gegebener Druckdifferenz bei einer Temperatur von 37°C über eine festgelegte Membranfläche durch die Membran tritt und das zur allgemeinen Vergleichbarkeit auf Flächeneinheit, Zeiteinheit und Druckeinheit normiert wird. Als Flüssigkeit zur Bestimmung der Ultrafiltrationsrate wird Wasser verwendet.

In der US 2008/0215247 A1 wird davon ausgegangen, dass der folgende Zusammenhang besteht:

$$Q_{UF} = TMP * K_{UF}$$

($Q_{UF}$ = Ultrafiltrationsrate, TMP = Transmembrandruck, $K_{UF}$ = Ultrafiltrationskoeffizient)

Ein linearer Zusammenhang scheint aber nur in einem gewissen Bereich des TMPs erfüllt zu sein. Die Funktion $Q_{UF}(TMP)$ wird zunächst abgeschätzt, indem der TMP sukzessive erhöht und die dadurch erzeugte Ultrafiltrationsrate gemessen wird. Ab einem bestimmten Wert hat eine Erhöhung des TMP eine immer geringere Steigerung der Ultrafiltrationsrate zur Folge. Man wählt daher den Kniepunkt (Tangentialpunkt) der Funktion $Q_{UF}(TMP)$ als Arbeitspunkt. Da sich der $K_{UF}$ während der Behandlung auf Grund der Systemänderung verschlechtert, wird in der WO 2006/011009 A2 gefordert, den Zusammenhang zwischen TMP und $Q_{UF}$ stündlich neu zu ermitteln.
In der EP 1175917 A1 wird als weiteres Konzept das Anpassen der Verhältnisse aus Prä- und Postdilution im Verlaufe der Behandlung beschrieben.

Ein ähnliches Vorgehen ist aus US 2005/0251086 bekannt, wobei ermittelt wird, ob sich durch das Anpassen des Verhältnisses aus Prä- und Postdilution eine erhöhte Clearance ergibt.

Ein weiterer Mechanismus berechnet anhand von Blutfluss, Hämatokrit Plasmaproteinkonzentration, Gewichtsverlust und Dialysatortyp die optimale Substitutionsrate. Diese kann automatisch während der Behandlung reduziert werden, wenn beispielsweise TMP-Alarme auftreten. TMP-Alarme sind eingebaute Routinen in modernen Dialysemaschinen oder Blutbehandlungseinheiten, wenn starke Verschlechterungen der Membraneigenschaften aufgrund eines dialysatseitigen Druckabfalls festgestellt werden.
Der Begriff "Substitutionsrate" definiert das Substitutionsvolumen, das pro Zeiteinheit verwendet wird. Typische Einheit ist hier ml/min, es wird aber auch in ml/h oder l/(Therapiedauer) angegeben.

Im Gegensatz zu den bekannten Methoden, die sich am Flüssigkeitstransport oder den anliegenden Drücken orientieren, um die Qualität einer konvektiven Behandlungsmethode wie z.B. der Hämodiafiltration zu verbessern, ist es Ziel der vorliegenden Erfindung, ein Online-Signal zu messen, das die tatsächliche Reinigungswirkung der Therapie zur Entfernung mittelmolekularer Substanzen aus dem Blut abbildet. Aufgrund dieser Messwerte soll eine optimierte Substitutionsrate ermittelt werden.

Substitutionsfluss, Substitutionsflussrate und Substitutionsrate sind hier synonym zu verstehen. Es wird hiermit immer das Volumen an Substituat beschrieben, das pro Zeiteinheit anfällt (typischerweise in ml/min). Das Substitutionsvolumen beschreibt die Gesamtmenge an Substituat und wird häufig auf eine gesamte Therapie (typischerweise 4 Stunden) bezogen.

Aufgabe der vorliegenden Erfindung ist die Bestimmung einer Flussrate einer Substitutionslösung, bei der eine durch einen vordefinierten Operationsmodus zu erreichende Reinigungsleistung für mittelmolekulare Substanzen erreicht wird. D.h. ausgehend von einem IST-Zustand der Reinigungsleistung für mindestens eine mittelmolekulare Substanz wird in Abhängigkeit vom gewählten Operationsmodus bestimmt, ob eine weitere Änderung, insbesondere Erhöhung des Substituatflusses eine noch akzeptable Steigerung der Reinigungsleistung bewirkt.

Diese Aufgabe wird durch die technische Lehre der unabhängigen Patentansprüche gelöst. Vorteilhafte Ausgestaltungen der Erfindung sind in den abhängigen Patentansprüchen, der Beschreibung, den Figuren sowie den Beispielen angegeben.

Es wurde in diesem Zusammenhang überraschend festgestellt, dass entgegen der allgemeinen Annahme eine Erhöhung des konvektiven Flusses sich nicht linear oder in einer ständigen Steigerung in der mittelmolekularen Reinigungsleistung niederschlägt. Dies hat zur Folge, dass der bisherige Ansatz, einen möglichst maximalen konvektiven Fluss zu erreichen, nicht zwangsläufig zu einer maximalen Behandlungsqualität führt und somit der konvektive Fluss als Maß für die Qualität der Behandlung nur bedingt aussagekräftig ist. Daher ist eine Anpassung der Behandlungsparameter allein auf einen maximalen Fluss hin nicht optimal.

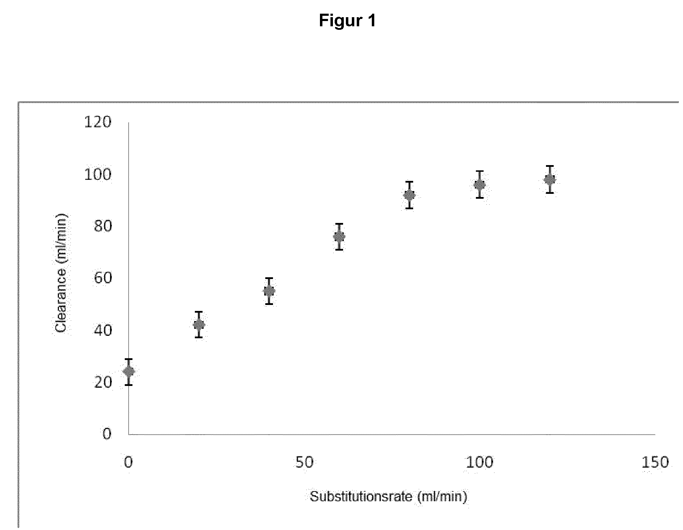

Die erfindungsgemäße Beobachtung stellt sich wie in der in Fig. 1 abgebildeten Messung der Reinigungsleistung in Abhängigkeit des Substitutionsflusses dar. Eine Erhöhung der Flussrate der Substitutionslösung führt anfänglich zu einer erhöhten Reinigung von mittelmolekularen Substanzen. Mit zunehmender Flussrate der Substitutionslösung kommt es zu einer Abflachung der Kurve. Die Reinigungsleistung strebt asymptotisch gegen einen Maximalwert, der durch die Filtercharakteristika, die Bauweise der Dialysemaschine, den physiologischen Zustand des Patienten und medizinische Grenzen der freien Parametenivahl charakterisiert ist. Es ist zu beachten, dass die Flussrate der Substitutionslösung nicht beliebig steigerbar ist, sondern die bekannten Abhängigkeiten zu Blutfluss, Hämatokrit und weiteren Behandlungsparametern aufweist, die in der Regel eine Obergrenze definieren. In der Regel sind Flussraten der Substitutionslösung zwischen 0 - 8 L pro Stunde realisierbar.

Dieser Verlauf läßt sich am besten durch eine Sättigungskurve darstellen. Da sich bei einer sehr geringen Flussrate der Substitutionslösung keine oder nur eine sehr geringe Reinigungsleistung einstellt. Ab einem gewissen Punkt wird bei einer für die Behandlungsbedingungen übermäßig hohen Substitutionsrate durch eine weitere Erhöhung der Flussrate ein gegenläufiger Effekt erzeugt. Die Membran wird derart beeinträchtigt, dass die Reinigungsleistung geringer wird. Einflussfaktoren sind hierbei zu kleine Filter, eine zu geringe Heparinisierung bzw. allgemeiner gesagt, eine zu geringe Antikoagulation, zu hohe Verhältnisse von Substituatfluss zu Blutfluss oder auch Immunreaktionen des Patienten, bei denen beispielsweise die Komplementaktivierung verstärkt wird.

Aufbauend auf dieser Messung der Reinigungsleistung von mittelmolekularen Substanzen in Abhängigkeit der Flussrate der Substitutionslösung ist es also möglich, behandlungs- und/oder patientenindividuell die geeignete Flussrate automatisch zu bestimmen. Um einen optimalen Fluss zu ermitteln, wird die Flussrate der Substitutionslösung erfindungsgemäß variiert und die erzeugte Signaländerung analysiert. Die Flussrate der Substitutionslösung kann auch indirekt über die Steuerung des TMPs und der Ultrafiltrationsrate eingestellt werden.

Unter "Flussrate der Substitutionslösung" wird das Volumen an Substitutionslösung bezeichnet, welche pro Zeiteinheit durch die Blutbehandlungseinheit und/oder durch den Sensor fließt.

Über ein solches Verfahren werden Messwerte erhalten, die, wenn man sie zueinander in Bezug setzt, einen Hinweis darauf geben, ob die Flussrate der Substitutionslösung nachgeregelt werden muss. Für die Notwendigkeit der Nachregelung kann ein Toleranzintervall festgelegt werden, innerhalb dessen Abweichungen von einem Erwartungswert für den jeweiligen Zeitpunkt toleriert werden, bei Überschreiten des Toleranzintervalls jedoch nachgeregelt werden soll. Der Erwartungswert ergibt sich aus einer Extrapolation der ersten Messwerte während einer Dialysesitzung. Zudem können Erfahrungswerte für den jeweiligen Bautyp des Dialysators, für den individuellen Dialysator und aus der Patientenhistorie in den Erwartungswert mit einfließen. Zur Variation der Flussrate der Substitutionslösung werden ein Minimalwert und ein Maximalwert festgelegt, zwischen denen über den ganzen Variationsbereich die Flussrate der Substitutionslösung variiert wird. Dies kann nach einem vorgegebenen Schema durchgeführt werden oder durch Eingabe eines Anwenders.

Abweichungen von den Grenzen der Toleranzintervalle und/oder des interpolierten Dialyseverlaufs können zum Anlass genommen werden, eine weitere Variation der Flussrate der Substitutionslösung vorzunehmen.

Zudem kann ein Zeitraum festgelegt werden, innerhalb dessen die Variation durchgeführt wird. Ebenfalls kann die Änderungsgeschwindigkeit festgelegt werden, mit der die Variation ausgeführt wird. Diese Variation kann mehrere Male während einer Dialysesitzung durchgeführt werden. Auch kann diese Variation des Flusses der Substitutionslösung in vordefinierten Zeitintervallen oder in frei wählbaren Zeitintervallen stattfinden. In bevorzugten Ausführungsformen sind die vordefinierten Zeitintervalle über den Verlauf der Behandlung gleich. In alternativen bevorzugten Ausführungsformen sind die vordefinierten Zeitintervalle am Anfang einer Dialysesitzung kürzer als an deren Ende, da für gewöhnlich gegen Ende eine geringere Variabilität des Messergebnisses auftritt. Somit werden die vordefinierten Zeitintervalle über den Verlauf der Behandlung zum Ende der Behandlung vorzugsweise größer. In einer weiteren bevorzugten Ausführrungsform werden die zum Ende der Behandlung vorzugsweise größer werdenden Zeitintervalle in Abhängigkeit der Änderungsgeschwindigkeit des Messsignals angepasst. Eine Umsetzungsmöglichkeit ist beispielsweise zu beobachten wie stark sich das Signal zwischen den letzten Messungen geändert hat und hieraus zu interpolieren wie lange es noch dauern wird bis ein bestimmter Änderungsgrad erreicht ist. Setzt man beispielsweise eine Grenze von 10%, so wird die Variation des Flusses der Substitutionslösung gestartet, sobald sich das Messsignal um 10% gegenüber dem Wert zum Zeitpunkt der letzten Variation geändert hat. Man kann diese Grenze auch über den Startwert zu Behandlungsbeginn definieren oder absolute Werte verwenden. Für die vorgenannten Verfahren gelten dieselben in der vorliegenden Anmeldung beschriebenen Ausführungsformen und Modifikationen.

Daher bezieht sich die vorliegende Erfindung auf ein Verfahren zur Bestimmung einer Flussrate einer Substitutionslösung, bei der eine Reinigungsleistung für mittelmolekulare Substanzen eines Tangentialflussfilters einer Blutbehandlungseinheit erreicht wird, die durch einen vorher festgelegten Bereich definiert wird.

Ferner umfasst eine weitere Ausführungsform des erfindungsgemäßen Verfahrens zur Bestimmung einer Flussrate einer Substitutionslösung bei der eine Reinigungsleistung für mittelmolekulare Substanzen eines Tangentialflussfilters einer Blutbehandlungseinheit erreicht wird, die folgenden Schritte:

a) Auswahl des vorher festgelegten Bereichs in Form eines Operationsmodus ausgewählt aus Optimum, Efficiency oder Economy;

b) Messung eines Signals, das durch mindestens eine mittelmolekulare Substanz im Dialysatfluss der Blutbehandlungseinheit erzeugt wird;

c) Variation der Flussrate der Substitutionslösung während der Messung nach b);

d) Weiterleitung der Flussraten der Substitutionslösung und der korrespondierenden Messwerte des Signals der mindestens einen mittelmolekularen Substanz im Dialysatfluss an eine zentrale Recheneinheit;
e) Korrelation der Flussraten der Substitutionslösung mit dem Signal der mindestens einen mittelmolekularen Substanz, wobei die Korrelation beinhaltet:

Bestimmen der Reinigungsleistung für die mindestens eine mittelmolekulare Substanz (K0) vor Änderung des Substituatflusses;
Bestimmen der Reinigungsleistung für die mindestens eine mittelmolekulare Substanz (K1) nach Änderung des Substituatflusses;
Bestimmen der Änderung des Substituatflusses in ml/min ($\Delta$S);
Berechnung der Änderung der Reinigungsleistung (P) für die mindestens eine mittelmolekulare Substanz in Abhängigkeit von der Änderung des Substituatflusses ($\Delta$S) gemäß:

$$\Delta K(0,1) = K1-K0$$

$$P= (\Delta K(0,1)/\Delta S)*100\%$$

wobei im Operationsmodus Optimum bestimmt wird, ob eine Erhöhung des Substituatflusses zumindest noch eine Steigerung von $10\% < P < 40\%$ bewirkt,
wobei im Operationsmodus Efficiency bestimmt wird, ob eine Erhöhung des Substituatflusses zumindest noch eine Steigerung von $40 \leq P < 70\%$ bewirkt,
wobei im Operationsmodus Economy bestimmt wird, ob eine Erhöhung des Substituatflusses zumindest noch eine Steigerung von $P \geq 70\%$ bewirkt, und

f) Ausgabe der aus e) ermittelten Werte entsprechend dem für den Operationsmodus festgelegten Bereich.

Sollten während der Behandlung Spülschritte zum Reinigen der Blutbehandlungseinheit durchgeführt werden, ist es bevorzugt, daß die Einstellung des gewünschten Substitutionsflusses unmittelbar nach einer vom Anwender oder einer automatisch durch die Maschine durchgeführten Reinigung des Dialysators vorgenommen wird.

Die Einstellung der Flussrate der Substitutionslösung hängt primär von der gewünschten Reinigungsleistung ab und kann auf verschiedene Parameter hin optimiert werden. Für diese spezifischen Parameter kann jeweils ein entsprechender Operationsmodus verwendet werden.

Primär wird mit dem erfindungsgemäßen Verfahren bestimmt, ob ausgehend von einem IST-Zustand der Reinigungsleistung für die mindestens eine mittelmolekulare Substanz eine weitere Änderung des Substituatflusses eine akzeptable Steigerung der Reinigungsleistung für die mindestens eine mittelmolekulare Substanz bewirkt. Was noch als akzeptable Steigerung bewertet wird, ergibt sich aus der Vorauswahl der Operationsmodi.

Erfindungsgemäß wird zunächst eine Reinigungsleistung K0 bei einem Substituatfluss S0 aufgenommen. Anschließend wird eine Änderung des Substituatflusses vorgenommen und die Reinigungsleistung K1 bei dem veränderten Substituatfluss S1 bestimmt.

Die Berechnung der Änderung der Reinigungsleistung (P) für die mindestens eine mittelmolekulare Substanz erfolgt in Abhängigkeit von der Änderung des Substituatflusses ($\Delta$S) gemäß:

$$\Delta K(0,1) = K1-K0$$

$$P= (\Delta K(0,1)/\Delta S)*100\%$$

Je nachdem, welcher Operationsmodus eingestellt wurde, ergibt sich für P ein Bereich, der für den eingestellten Operationsmodus angestrebt wird.

Erfindungsgemäß können verschiedene Operationsmodi der Substitutionsflussregelung definiert werden:

a) Optimum Modus
b) Efficiency Modus
c) Eco Modus

Erfindungsgemäß kann der Operationsmodus aber auch vom Anwender selbst definiert werden.

Im Optimum Modus wird eine optimale oder nahezu optimale Reinigungsleistung der mittelmolekularen Substanzen erzielt, ohne dass dabei der Verbrauch an Substitutionsflüssigkeit im Vordergrund steht. Dieser Modus zeichnet sich durch einen Bereich aus, der durch eine obere und eine untere Grenze für die Änderung der Reinigungsleistung (P) definiert wird. Die untere Grenze der Änderung der Reinigungsleistung (P) ist dadurch gekennzeichnet, dass auch Erhöhungen des Substituatflusses, die nur noch geringe Steigerungen der Reinigungsleistung der mittelmolekularen Substanz bewirken, noch toleriert werden. D.h., solange die Erhöhungen des Substituatflusses noch zu einer Steigerung der Reinigungsleistung der mittelmolekularen Substanz führen, die über der unteren Grenze für die Änderung der Reinigungsleistung (P) liegen, ist der Bereich des Optimum Modus noch nicht verlassen. In diesem Bereich sollen vorzugsweise die Steigerungen des Substituatflusses nur noch in kleinen Schritten von weniger als 10 ml/min, bevorzugt weniger als 7 ml/min und weiter bevorzugt weniger als 5 ml/min erfolgen.

Ebenso wird der Optimum Modus durch einen oberen Grenzwert für die Änderung der Reinigungsleistung (P) definiert. Dieser obere Grenzwert ist notwendig, da es das Ziel im Optimum Modus ist, eine optimale oder nahezu optimale Reinigungsleistung der mittelmolekularen Substanz zu erzielen, unabhängig vom Verbrauch der Substitutionsflüssigkeit. Die Kurve für die Reinigungsleistung der mittelmolekularen Substanz entspricht im Kern einer Sättigungskurve. Im vorderen Teil der Kurve, d.h. bei noch niedrigen Substituatflussraten, können durch kleine Änderungen der Substituatflussrate noch große Änderungen der Reinigungsleistung (P) erzielt werden. Wenn nun im Optimum Modus eine Änderung der Reinigungsleistung (P) gemessen wird, die den oberen Grenzwert überschreitet, dann ist dies ein Anzeichen dafür, dass die optimale Reinigungsleistung noch lange nicht erreicht ist und daher eine weitere Veränderung bzw. Erhöhung des Substituatflusses nötig ist.

Bevorzugterweise wird im Optimum Modus angestrebt, dass eine Erhöhung des Substituatflusses zumindest noch eine Steigerung von 10% < P < 40% bewirkt. Die untere Grenze für den Wert P ist variabel und beträgt entweder 2%, 4%, 6%, 8%, 10%, 12%, 14%, 16%, 18%, 20%, 22%, 24%, 26%, 28% oder 30%. Die obere Grenze ist ebenso variabel entspricht aber mindestens der unteren Grenze und beträgt entweder 20%, 22%, 24%, 26%, 28%, 30%, 32%, 34%, 36%, 38%, 40%, 42%, 44%, 46%, 48% oder 50%. Die unteren und oberen Grenzwerte sind frei miteinander kombinierbar, solange der obere Grenzwert ≥ dem unteren Grenzwert und vorzugsweise > als der untere Grenzwert ist. Alternative Grenzbereiche sind: 12% < P < 40%, 14% < P < 40%, 16% < P < 40%, 18% < P < 40%, 20% < P < 40%, 12% < P < 45%, 14% < P < 45%, 16% < P < 45%, 18% < P < 45%, 20% < P < 45%, 12% < P < 50%, 14% < P < 50%, 16% < P < 50%, 18% < P < 50%, 20% < P < 50%, 10% < P < 42%, 10% < P < 44%, 10% < P < 46%, 10% < P < 48%, 10% < P < 50%, 10% < P < 38%, 10% < P < 36%, 10% < P < 34%, 10% < P < 32%, 10% < P < 30%, 15% < P < 42%, 15% < P < 44%, 15% < P < 46%, 15% < P < 48%, 15% < P < 50%, 15% < P < 38%, 15% < P < 36%, 15% < P < 34%, 15% < P < 32% oder 15% < P < 30%.

Allerdings wird in diesem Operationsmodus überproportional viel Substitutionslösung verbraucht. Substitutionsraten liegen beim Optimum Modus typischerweise im Bereich über 100 ml/min, wobei dieser Wert aber nur als ungefährer Wert gesehen werden kann. Die Grenze ist hier durch das Verhältnis aus Blutfluss zur Substitutionsrate gesetzt. Wird das Blut durch einen zu hohen Flüssigkeitsentzug im Filter zu sehr aufkonzentriert, wird der Filter verstopfen. Als Faustformel gilt hier, dass die Substitutionsrate ein Drittel des Blutflusses ausmachen kann. Der Automatismus erkennt diesen Punkt durch Überwachung der blut- sowie dialysatseitigen Drücke, sowie durch die Überwachung des Hämatokrits. Soll die Substitutionsrate trotz Erreichen der Grenze weiter erhöht werden, so ist dies möglich indem zusätzlich die Prädilution gestartet wird und so das Blut vor dem Filter verdünnt wird. Dies ermöglicht das weitere Erhöhen der Substitutionsrate.

Dieser Modus wird besonders bevorzugt, wenn aufgrund auffällig hoher Messwerte mittelmolekularer Substanzen, durch ein akutes Nierenversagen (beispielsweise bei Multiorganversagen oder nach einer Operation) oder bei akuten Vergiftungen es medizinisch indiziert ist, so schnell wie möglich eine weitestgehende Reinigung des Blutes u.a. von mittelmolekularer Substanzen zu erreichen. Die Kostenfrage tritt hierbei in den Hintergrund.

Der Efficiency Modus bildet einen mittleren Bereich des Flusses der Substitutionslösung ab. In diesem Bereich wird der bestmögliche Kompromiss aus Wirtschaftlichkeit und optimaler Reinigungsleistung der mittelmolekularen Substanzen gesucht. Typischerweise liegt dieser Punkt bei Substitutionsraten zwischen 50 ml/min und 100 ml/min (im Falle der Postdilution), wobei es sich aber auch hier nur um einen ungefähren Bereich handelt, der sich je nach Begebenheiten ändern kann.

Auch der Efficiency Modus zeichnet sich durch einen Bereich aus, der durch eine obere und eine untere Grenze für die Änderung der Reinigungsleistung (P) definiert wird. Im Gegensatz zu dem Optimum Modus liegt sowohl die untere Grenze als auch die obere Grenze für die Änderung der Reinigungsleistung (P) um einiges höher. Ziel dieses Modus ist es nicht, eine optimale Reinigungsleistung zu erzielen, sondern mit einem noch vertretbaren Verbrauch an Substituatflüssigkeit, das bestmögliche Ergebnis zu erzielen. Die untere Grenze der Änderung der Reinigungsleistung (P) ist somit dadurch gekennzeichnet, dass Erhöhungen des Substituatflusses, die nur noch geringe Steigerungen der Reinigungsleistung der mittelmolekularen Substanz bewirken, nicht mehr effizient sind und daher aus dem Bereich herausfallen.

Der obere Grenzwert für die Änderung der Reinigungsleistung (P) ist notwendig, da es das Ziel im Efficiency Modus ist, einen Kompromiss zwischen Substituatsflüssigkeitsverbrauch und Reinigungsleistung der mittelmolekularen Substanz zu erzielen. Wenn nun im Efficiency Modus eine Änderung der Reinigungsleistung (P) gemessen wird, die den oberen Grenzwert überschreitet, dann ist dies ein Anzeichen dafür, dass die Reinigungsleistung noch mit einem überschaubaren Verbrauch an Substitutionsflüssigkeit gesteigert werden könnte.

Bevorzugterweise wird im Efficiency Modus angestrebt, dass eine Erhöhung des Substituatflusses zumindest noch eine Steigerung von 40% ≤ P < 70% bewirkt. Die untere Grenze für den Wert P ist variabel und beträgt entweder 32%, 34%, 36%, 38%, 40%, 42%, 44%, 46%, 48%, 50%, 52%, 54%, 56%, 58% oder 60%. Die obere Grenze ist ebenso variabel entspricht aber mindestens der unteren Grenze und beträgt entweder 60%, 62%, 64%, 66%, 68%, 70%, 72%, 74%, 76%, 78%, 70%, 72%, 74%, 76%, 78% oder 80%. Die unteren und oberen Grenzwerte sind frei miteinander kombinierbar, solange der obere Grenzwert ≥ dem unteren Grenzwert ist. Alternative Grenzbereiche sind: 42%≤ P < 65%, 44% ≤ P < 65%, 46% ≤ P < 65%, 48% ≤ P < 65%, 50% ≤ P < 65%, 38% ≤ P < 65%, 36% ≤ P < 65%, 34% ≤ P < 65%, 32% ≤ P < 65%, 30% ≤ P < 65%, 42% ≤ P < 70%, 44% ≤ P < 70%, 46% ≤ P < 70%, 48% ≤ P < 70%, 50% ≤ P < 70%, 38% ≤ P < 70%, 36% ≤ P < 70%, 34% ≤ P < 70%, 32% ≤ P < 70%, 30% ≤ P < 70%, 42% ≤ P < 75%, 44% ≤ P < 75%, 46% ≤ P < 75%, 48% ≤ P < 75%, 50% ≤ P < 75%, 38% ≤ P < 75%, 36% ≤ P < 75%, 34% ≤ P < 75%, 32% ≤ P < 75%, 30% ≤ P < 75%, 35% ≤ P < 72%, 35% ≤ P < 74%, 35% ≤ P < 76%, 35% ≤ P < 78%, 35% ≤ P < 80%, 35% ≤ P < 68%, 35% ≤ P < 66%, 35% ≤ P < 64%, 35% ≤ P < 62%, 35% ≤ P < 60%, 40% ≤ P < 72%, 40% ≤ P < 74%, 40% ≤ P < 76%, 40% ≤ P < 78%, 40% ≤ P < 80%, 40% ≤ P < 68%, 40% ≤ P<66%, 40% ≤ P < 64%, 40% ≤ P < 62%, 40% ≤ P < 60%, 45% ≤ P < 72%, 45% ≤ P < 74%, 45% ≤ P < 76%, 45% ≤ P < 78%, 45% ≤ P < 80%, 45% ≤ P < 68%, 45% ≤ P < 66%, 45% ≤ P < 64%, 45% ≤ P < 62% oder 45% ≤ P < 60%.

Für die Mehrzahl der Anwendungen wird dieser Modus besonders bevorzugt sein.

Ferner ist bevorzugt, wenn sich die Obergrenze von P des Operationsmodus Optimum (z.B. 40%) an die Untergrenze von P im Operationsmodus Efficiency anschließt und sich die Obergrenze von P des Operationsmodus Efficiency (z.B. 70%) an die Untergrenze von P im Operationsmodus Economy anschließt.

Eine weitere Herangehensweise zur Bestimmung dieses Punktes läßt sich theoretisch folgendermaßen herleiten: wie bei vielen begrenzten Ressourcen ist davon auszugehen, daß sich bei der Dialyse der Einsatz wirtschaftlicher Mittel im Verhältnis zum erzielten Erfolg (der Dialyseleistung) *cum grano salis* am Gesetz des Grenzwertnutzens orientiert. Dies ist ein volkswirtschaftlich etabliertes Modell zur Ermittlung des optimalen Ressourceneinsatzes. Hierbei ergibt der Zusammenhang zwischen Mitteleinsatz und erzieltem Erfolg eine sigmoide Kurve, wie sie zuerst 1838 von Pierre Verhulst auf der Basis des Bevölkerungsgesetzes von Malthus entwickelt wurde:

$$\frac{dP}{dt} = kP(C - P)$$

(P: Populationsgröße; t: Zeit; k: Konstante proportional zur Wachstumsrate; C: maximale Kapazität).

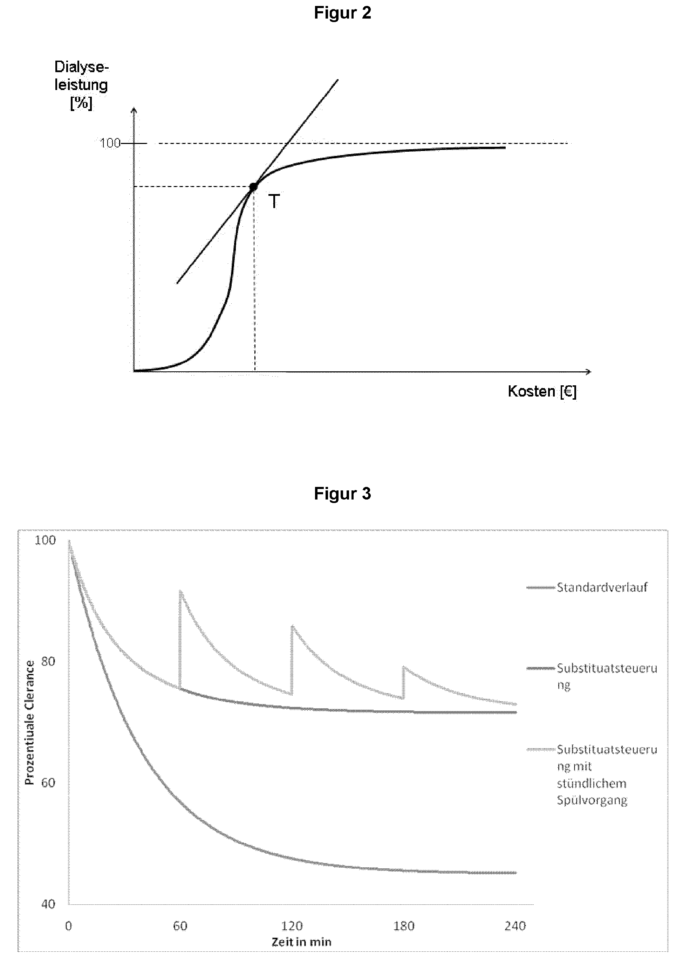

Der Optimumswert, hier die höchste Dialyseeffizienz, befindet sich demnach an dem Tangentialpunkt T der sigmoiden Kurve. In Fig. 2 ist dieser Punkt mit T bezeichnet und eine fiktive Tangente wurde an diesen Punkt gelegt. Um für einen Dialyseprozess diesen T-Punkt zumindest annäherungsweise zu ermitteln, bedarf es einer Ermittlung der Kosten und ein belastbares Maß für die Dialyseeffizienz.

Die Kosten ergeben sich aus einer Funktion

$$K = F + I\,(t) + D\,(v,\,t)$$

Hierbei sind K die Gesamtkosten, F die proportionalen Fixkosten einer Dialysestation, t die Dauer einer Dialysebehandlung, I (t) die von t abhängigen Infrastrukturkosten für z.B. ein Dialysebett und die Personalkosten, v die Flussrate der Substitutionslösung und D die von und t abhängigen Kosten für den Verbrauch der Substitutionslösung.

Der Eco Modus (Economy Modus) zeichnet sich dadurch aus, daß vergleichsweise wenig Substitutionslösung verbraucht wird, um ein qualitativ immer noch gutes Reinigungsergebnis für die mittelmolekularen Substanzen zu erzielen. Hier steht der Verbrauch an Substitutionsflüssigkeit im Vordergrund. Dieser Modus zeichnet sich durch einen Bereich aus, der durch eine untere Grenze für die Änderung der Reinigungsleistung (P) definiert wird. Die untere Grenze der Änderung der Reinigungsleistung (P) ist dadurch gekennzeichnet, dass nur Erhöhungen des Substituatflusses, die substantielle Steigerungen der Reinigungsleistung der mittelmolekularen Substanz bewirken, toleriert werden.

Bevorzugterweise wird im Eco Modus angestrebt, dass eine Erhöhung des Substituatflusses zumindest noch eine Steigerung von P ≥ 70% bewirkt. Die untere Grenze für den Wert P ist variabel und beträgt entweder 62%, 64%, 66%, 68%, 70%, 72%, 74%, 76%, 78%, 80%, 82%, 84%, 86%, 88% oder 90%.

Eine beträchtliche Menge an Substitutionslösung kann eingespart werden. Wenn der Schwerpunkt der Dialyse auf einem möglichst wirtschaftlichem Betrieb liegt, ist der Eco Modus besonders bevorzugt. Typische Flussraten liegen hier bei 30 ml/min - 50 ml/min oder darunter, wobei sich auch hier die Bereiche nach oben bzw. nach unten verschieben lassen.

In einer weiteren Ausführungsform des erfindungsgemäßen Verfahrens zur Bestimmung einer Flussrate einer Substitutionslösung bei der eine Reinigungsleistung für mittelmolekulare Substanzen eines Tangentialflussfilters einer Blutbehandlungseinheit erreicht werden, umfasst die folgenden Schritte:

a) Auswahl des vorher festgelegten Bereichs in Form eines Operationsmodus ausgewählt aus Optimum, Efficiency oder Economy;
b) Messung eines Signals, das durch mindestens eine mittelmolekulare Substanz im Dialysatfluss der Blutbehandlungseinheit erzeugt wird;
c) Variation der Flussrate der Substitutionslösung während der Messung nach b);
d) Weiterleitung der Flussraten der Substitutionslösung und der korrespondierenden Messwerte des Signals der mindestens einen mittelmolekularen Substanz im Dialysatfluss an eine zentrale Recheneinheit;
e) Korrelation der Flussraten der Substitutionslösung mit dem Signal der mindestens einen mittelmolekularen Substanz, wobei die Korrelation beinhaltet:

Bestimmen der Reinigungsleistung für die mindestens eine mittelmolekulare Substanz (K0) vor Änderung des Substituatflusses;
Bestimmen der Reinigungsleistung für die mindestens eine mittelmolekulare Substanz (K1) nach einer ersten Änderung des Substituatflusses;
Bestimmen der Reinigungsleistung für die mindestens eine mittelmolekulare Substanz (K2) nach einer zweiten Änderung des Substituatflusses;
Berechnung der Änderung der Reinigungsleistung (P) für die mindestens eine mittelmolekulare Substanz gemäß:

$$\Delta K(0,1) = K1\text{-}K0$$

$$\Delta K(1,2) = K2\text{-}K1$$

$$P = (\Delta K(1,2) / \Delta K(0,1))*100\%$$

wobei im Operationsmodus Optimum bestimmt wird, ob eine Erhöhung des Substituatflusses zumindest noch eine Steigerung von P > 25% bewirkt,
wobei im Operationsmodus Efficiency bestimmt wird, ob eine Erhöhung des Substituatflusses zumindest noch eine Steigerung von P > 65% bewirkt,
wobei im Operationsmodus Economy bestimmt wird, ob eine Erhöhung des Substituatflusses zumindest noch eine Steigerung von P > 85% bewirkt, und

f) Ausgabe der aus e) ermittelten Werte entsprechend dem für den Operationsmodus festgelegten Bereich.

In dieser Ausführungsform wird zunächst die aktuelle Reinigungsleitung für die mindestens eine mittelmolekulare Substanz gemessen. Danach wird eine erste Steigerung der Substitutionsflussrate durchgeführt, die eine erste Veränderung der Reinigungsleistung für die mindestens eine mittelmolekulare Substanz zur Folge hat. In einem weiteren Schritt wird eine zweite Steigerung der Substitutionsflussrate durchgeführt, die eine zweite Veränderung der Reinigungsleistung für die mindestens eine mittelmolekulare Substanz bewirkt. Ausgehend hiervon wird nun bestimmt, ob die zweite Veränderung der Reinigungsleistung gegenüber der ersten Veränderung in einem akzeptablen Rahmen liegt. Was noch als akzeptable Steigerung bewertet wird, ergibt sich aus der Vorauswahl der Operationsmodi.

Erfindungsgemäß wird zunächst eine Reinigungsleistung K0 bei einem Substituatfluss S0 aufgenommen. Anschließend wird eine erste Änderung des Substituatflusses vorgenommen und die Reinigungsleistung K1 bei dem veränderten Substituatfluss S1 bestimmt. In einem zweiten Schritt wird eine zweite Änderung des Substituaflusses vorgenommen und die Reinigungsleistung K2 bei dem veränderten Substituatfluss S2 bestimmt.

Die Berechnung der Änderung der Reinigungsleistung (P) für die mindestens eine mittelmolekulare Substanz

erfolgt gemäß:

$$\Delta K(0,1) = K1-K0$$

$$\Delta K(1,2) = K2-K1$$

$$P = (\Delta K(1,2) / \Delta K(0,1))*100\%$$

Je nachdem, welcher Operationsmodus eingestellt wurde, ergibt sich für P ein Bereich, der für den eingestellten Operationsmodus angestrebt wird.

Es ist für den Fachmann ersichtlich, dass nach obiger Formel die zweite Steigerung der Substitutionsflussrate identisch mit der ersten Steigerung sein muss, um reine Unterschiede auszuschließen, welche rein auf unterschiedlichen Steigerungen beruhen. Allerdings ist es auch möglich, dass die Veränderungen der ersten und zweiten Substitutionsflussraten voneinander abweichen. In diesem Fall müssen $\Delta K(0,1)$ = K1-K0 als auch $\Delta K(1,2)$ = K2-K1 auf ihre jeweilige Substitutionsflussrate relativiert werden. Zum Beispiel könnte die erste Steigerung der Substitutionsflussrate mit einem Wert von 10 ml/min durchgeführt worden sein und die zweite Steigerung der Substitutionsflussrate mit 5 ml/min. Um den oben genannten Effekt zu vermeiden, müsste nun der Wert aus $\Delta K(0,1)$ durch 10 dividiert werden und der Wert aus $\Delta K(1,2)$ durch 5. D.h. teilt man $\Delta K(0,1)$ durch die erste Steigerung und $\Delta K(1,2)$ durch die zweite Steigerung, so können auch unterschiedliche Steigerungen verwendet werden und $\Delta K(0,1)$ und $\Delta K(1,2)$ lassen sich dennoch weiterhin in Relation setzen.

Erfindungsgemäß können verschiedene Operationsmodi der Substitutionsflussregelung definiert werden:

a) Optimum Modus
b) Efficiency Modus
c) Eco Modus

Erfindungsgemäß kann der Operationsmodus aber auch vom Anwender selbst definiert werden.

In dieser Ausführungsform wird im Optimum Modus eine optimale oder nahezu optimale Reinigungsleistung der mittelmolekularen Substanzen erzielt, ohne dass dabei der Verbrauch an Substitutionsflüssigkeit im Vordergrund steht. Dieser Modus zeichnet sich durch einen Bereich aus, der durch eine untere Grenze für die Änderung der Reinigungsleistung (P) definiert wird. Die untere Grenze der Änderung der Reinigungsleistung (P) ist dadurch gekennzeichnet, dass auch Erhöhungen des Substituatflusses, die nur noch geringe Steigerungen der Reinigungsleistung der mittelmolekularen Substanz bewirken, noch toleriert werden. D.h., solange die Erhöhungen des Substituatflusses noch zu einer Steigerung der Reinigungsleistung der mittelmolekularen Substanz führen, die über der unteren Grenze für die Änderung der Reinigungsleistung (P) liegen, ist der Bereich des Optimum Modus noch nicht verlassen. In diesem Bereich sollen vorzugsweise die Steigerungen des Substituatflusses nur noch in kleinen Schritten von weniger als 10 ml/min, bevorzugt weniger als 7 ml/min und weiter bevorzugt weniger als 5 ml/min erfolgen.

Bevorzugterweise wird im Optimum Modus angestrebt, dass eine Erhöhung des Substituatflusses zumindest noch eine Steigerung von P > 25% bewirkt. Die Grenze für den Wert P ist variabel und beträgt entweder 2%, 4%, 6%, 8%, 10%, 12%, 14%, 16%, 18%, 20%, 22%, 24%, 26%, 28% oder 30.

Allerdings wird in diesem Operationsmodus überproportional viel Substitutionslösung verbraucht. Substitutionsraten liegen beim Optimum Modus typischerweise im Bereich über 100 ml/min, wobei dieser Wert aber nur als ungefährer Wert gesehen werden kann. Die Grenze ist hier durch das Verhältnis aus Blutfluss zur Substitutionsrate gesetzt. Wird das Blut durch einen zu hohen Flüssigkeitsentzug im Filter zu sehr aufkonzentriert, wird der Filter verstopfen. Als Faustformel gilt hier, dass die Substitutionsrate ein Drittel des Blutflusses ausmachen kann. Der Automatismus erkennt diesen Punkt durch Überwachung der blut- sowie dialysatseitigen Drücke, sowie durch die Überwachung des Hämatokrits. Soll die Substitutionsrate trotz Erreichen der Grenze weiter erhöht werden, so ist dies möglich indem zusätzlich die Prädilution gestartet wird und so das Blut vor dem Filter verdünnt wird. Dies ermöglicht das weitere Erhöhen der Substitutionsrate.

Dieser Modus wird besonders bevorzugt, wenn aufgrund auffällig hoher Messwerte mittelmolekularer Substanzen, durch ein akutes Nierenversagen (beispielsweise bei Multiorganversagen oder nach einer Operation) oder bei akuten Vergiftungen es medizinisch indiziert ist, so schnell wie möglich eine weitestgehende Reinigung des Blutes u.a. von mittelmolekularer Substanzen zu erreichen. Die Kostenfrage tritt hierbei in den Hintergrund.

In dieser Ausführungsform bildet der Efficiency Modus einen mittleren Bereich des Flusses der Substitutionslösung ab. In diesem Bereich wird der bestmögliche Kompromiss aus Wirtschaftlichkeit und optimaler Reinigungsleistung der mittelmolekularen Substanzen gesucht. Typischerweise liegt dieser Punkt bei Substitutionsraten zwischen 50 ml/min und 100 ml/min (im Falle der Postdilution), wobei es sich aber auch hier nur um einen ungefähren Bereich handelt, der sich je nach Begebenheiten ändern kann.

Der Efficiency Modus zeichnet sich durch einen Bereich aus, der durch eine untere Grenze für die Änderung der Reinigungsleistung (P) definiert wird. Im Gegensatz zu dem Optimum Modus liegt hier die Grenze für die Änderung der Reinigungsleistung (P) um einiges höher. Ziel dieses Modus ist es nicht, eine optimale Reinigungsleistung zu erzielen, sondern mit einem noch vertretbaren Verbrauch an Substituatsflüssigkeit, das bestmögliche Ergebnis zu erzielen. Die untere Grenze der Änderung der Reinigungsleistung (P) ist somit dadurch gekennzeichnet, dass Erhöhungen des Substituatflusses, die nur noch geringe Steigerungen der Reinigungsleistung der mittelmolekularen Substanz bewirken, nicht mehr effizient sind und daher aus dem Bereich herausfallen.

Bevorzugterweise wird im Efficiency Modus angestrebt, dass eine Erhöhung des Substituatflusses zumindest noch eine Steigerung von P > 65% bewirkt. Die untere Grenze für den Wert P ist variabel und beträgt entweder 32%, 34%, 36%, 38%, 40%, 42%, 44%, 46%, 48%, 50%, 52%, 54%, 56%, 58%, 60%, 62%, 64%, 66%, 68%, 70%, 72%, 74%, 76%, 78%, 70%, 72%, 74%, 76%, 78% oder 80.

In dieser Ausführungsform zeichnet sich der Eco Modus (Economy Modus) dadurch aus, dass vergleichsweise wenig Substitutionslösung verbraucht wird, um ein qualitativ immer noch gutes Reinigungsergebnis für die mittelmolekularen Substanzen zu erzielen. Hier steht der Verbrauch an Substitutionsflüssigkeit im Vordergrund. Dieser Modus zeichnet sich durch einen Bereich aus, der durch eine untere Grenze für die Änderung der Reinigungsleistung (P) definiert wird. Die untere Grenze der Änderung der Reinigungsleistung (P) ist dadurch gekennzeichnet, dass nur Erhöhungen des Substituatflusses, die substantielle Steigerungen der Reinigungsleistung der mittelmolekularen Substanz bewirken, toleriert werden.

Bevorzugterweise wird im Eco Modus angestrebt, dass eine Erhöhung des Substituatflusses zumindest noch eine Steigerung von P > 85% bewirkt. Die untere Grenze für den Wert P ist variabel und beträgt entweder 80%, 82%, 84%, 86%, 88%, 90%, 92%, 94%, 96%, 98% oder 100%.

Eine beträchtliche Menge an Substitutionslösung kann eingespart werden. Wenn der Schwerpunkt der Dialyse auf einem möglichst wirtschaftlichem Betrieb liegt, ist der Eco Modus besonders bevorzugt. Typische Flussraten liegen hier bei 30 ml/min - 50 ml/min oder darunter, wobei sich auch hier die Bereiche nach oben bzw. nach unten verschieben lassen.

Wenn eine maximale mittelmolekulare Reinigungsleistung gewünscht oder erforderlich ist, kann der Substitutionsfluss im Optimum Modus gefahren werden. Die Bestimmung der maximalen mittelmolekularen Reinigungsleistung ist durch die erfindungsgemäße Beobachtung gekennzeichnet, dass es einen Punkt gibt, an dem eine weitere Erhöhung der Flussrate der Substitutionslösung zu keiner oder nur noch einer geringfügigen weiteren Steigerung der mittelmolekularen Reinigungsleistung führt, oder, die weitere Erhöhung der Flussrate der Substitutionslösung eine Verschlechterung der mittelmolekularen Reinigungsleistung bedingt. Somit bedient sich das Verfahren dieser Beobachtung, indem die Flussrate der Substitutionslösung kontinuierlich durch Anhebung oder Absenkung variiert wird und gleichzeitig, das Signal einer mittelmolekularen Substanz im Dialysatausfluss der Blutbehandlungseinheit gemessen wird. Der Punkt der maximalen mittelmolekularen Reinigungsleistung ist damit der Punkt, an dem eine weitere Steigerung der Flussrate zu keiner bzw. nur noch minimalen weiteren Absenkung der Absorbanz führt, sondern zu einer Steigerung der Absorbanz der mindestens einen mittelmolekularen Substanz. Die Einstellung der Flussrate der Substitutionslösung auf diesen Wert ermöglicht eine optimale oder nahezu optimale mittelmolekulare Reinigungsleistung.

Wenn ein Kompromiss aus maximaler mittelmolekularer Reinigungsleistung und der Wirtschaftlichkeit der Dialysetherapie gewünscht oder erforderlich ist, kann der Substitutionsfluss im Efficiency Modus gefahren werden.

Die Bestimmung der effizienten mittelmolekularen Reinigungsleistung ist durch die erfindungsgemäße Beobachtung gekennzeichnet, dass es Bereiche gibt, in denen eine Erhöhung der Flussrate der Substitutionslösung noch nicht in einer Verschlechterung der mittelmolekularen Reinigungsleistung resultiert, aber jegliche weitere Steigerung nur einen sehr geringen Effekt auf die mittelmolekulare Reinigungsleistung hat. Somit ist es möglich, eine Steigung der mittelmolekularen Reinigungsleistung zu definieren, die als Minimum definiert wird. In diesem Bereich wird es für nicht weiter effizient erachtet, die Flussrate der Substitutionslösung weiter zu erhöhen. Dieses Vorgehen optimiert den Verbrauch an Substitutionslösung zur Erreichung einer guten mittelmolekularen Reinigungsleistung.

In einer weiteren Ausführungsform kann es auch vorteilhaft sein, eine mittelmolekulare Reinigungsleistung anzustreben, die unter ökonomischen Gesichtspunkten gewählt wird. Hierbei liegt das Augenmerk auf dem Erzielen einer medizinisch noch befriedigenden mittelmolekularen Reinigungsleistung bei möglichst geringem Verbrauch an Substitutionslösung.

Die Bestimmung der ökonomischen mittelmolekularen Reinigungsleistung ist dadurch gekennzeichnet, dass ein Reinigungsminimum angegeben wird, welches erreicht werden soll. Daraufhin wird der Behandlungsverlauf so angepasst, dass dieses Ziel erreicht wird, aber keine Substitutionslösung aufgewendet werden muss, um dieses noch weiter zu steigern. Hierzu können verschiedene Wege eingeschlagen werden. Zum einen kann versucht werden, dieses Ziel so schnell wie möglich zu erreichen und im Anschluss daran kann die Substitution auf ein Minimum reduziert werden. Alternativ kann auch eine Vorhersage für das nötige Substitutionsvolumen getroffen werden und dann dieses bis zum Ende der Therapie umgesetzt werden. Hierbei kann sich auch ergeben, dass die Substitution vorrübergehend oder endgültig auf sehr kleine Werte oder sogar auf Null reduziert wird.

In bevorzugten Ausführungsformen kann um jede für einen jeweiligen Operationsmodus erfindungsgemäß ermittelte Flussrate der Substitutionslösung, dem Zentralwert, ein Toleranzintervall festgelegt werden. Wenn sich eine eingestellte oder gemessene Flussrate innerhalb dieses Toleranzintervalls befindet, soll sie daher als diesem Operationsmodus entsprechend gelten. Die Größe der Toleranzintervalle können für die verschiedenen Operationsmodi gleich sein oder individuell gestaltet sein. Sie können entweder vom Hersteller der Dialysemaschine vorgegeben sein, von der zentralen Recheneinheit vorgeschlagen werden oder vom Anwender individuell festgelegt werden. Hierbei können die Toleranzintervalle über den Bereich der realistisch wählbaren Flussraten der Substitutionslösung für die jeweiligen Operationsmodi aneinander angrenzen, sich überschneiden oder zwischen einander Lücken aufweisen. Bei sich überschneidenden Toleranzintervallen zweier benachbarter Operationsmodi soll für Flussraten im Überschneidungsbereich gelten, daß diese Flussraten mit beiden Operationsmodi kompatibel sind. Die Größe der Toleranzintervalle kann in Absolutwerten der Flussrate der Substitutionslösung angegeben werden, oder aber in einer prozentualen Abweichung der Flussrate von dem zuvor ermittelten Zentralwert für einen Operationsmodus. Bevorzugt wird beispielsweise ein Toleranzintervall von ± 25% um den Zentralwert eines Operationsmodus, bevorzugter von ± 15%, weiter bevorzugt ± 10% und am meisten bevorzugt von ± 5%.

Bei einem auffälligen Verlauf eines dialyserelevanten Parameters während einer Dialysesitzung, der sich in der Online-Überwachung des Substitutionsflusses bemerkbar macht, kann dieser variiert werden, um die Reinigungsleistung für mittelmolekulare Substanzen zu optimieren.

Bei derart auffälligen Messwerten kann von der zentralen Recheneinheit die Empfehlung ergehen, den Operationsmodus zu ändern, oder eine solche Änderung kann automatisch vorgenommen werden. Ein derartiger dialyserelevanter Parameter und/oder auffälliger Messwert kann das besagte Signal, der Blutdruck des Patienten, der Flüssigkeitsstatus des Patienten oder die niedermolekulare Reinigungsleistung sein.
Die erfindungsgemäßen Verfahren ermöglichen somit, dass bei einem auffälligen Verlauf eines dialyserelevanten Parameters während einer Dialysesitzung ein Optimierungsprozess des Substitutionsflusses gestartet wird, wobei der dialyserelevante Parameter das besagte Signal, der Blutdruck des Patienten, der Flüssigkeitsstatus des Patienten oder die niedermolekulare Reinigungsleistung sein kann.

Eine weitere erfindungsgemäße Beobachtung stellt sich wie in Fig. 3 gezeigt dar. Über den Verlauf der Dialyse nimmt die Reinigungsleistung in Form der mittelmolekularen Reinigungsleistung stetig ab und pendelt sich auf einem niedrigeren Niveau ein. Dies ist darauf zurückzuführen, dass sich die Membraneigenschaften im Verlauf der Dialyse verändern, z.B. die Membran sich mit Proteinen zusetzt. Wie bereits beschrieben, sehen bisherige Ansätze vor, einen möglichst maximalen konvektiven Fluss zu erreichen, wodurch sich die Membraneigenschaften aber noch schneller verändern (Standardverlauf). Eine **Substitutionsrate,** die nicht auf eine reine maximale Flussrate gerichtet ist, sondern durch Messung der mittelmolekularen Reinigungsleistung optimal gesteuert wird, zeigt über den Verlauf einer Dialyse eine durchgehend höhere Reinigungsleistung. Man spricht daher von einer Substitutionssteuerung. Diese Reinigungsleistung kann noch weiter gesteigert werden, indem intermittierend jeweils ein Spülvorgang durchgeführt wird (Substitutionssteuerung mit Spülvorgang).

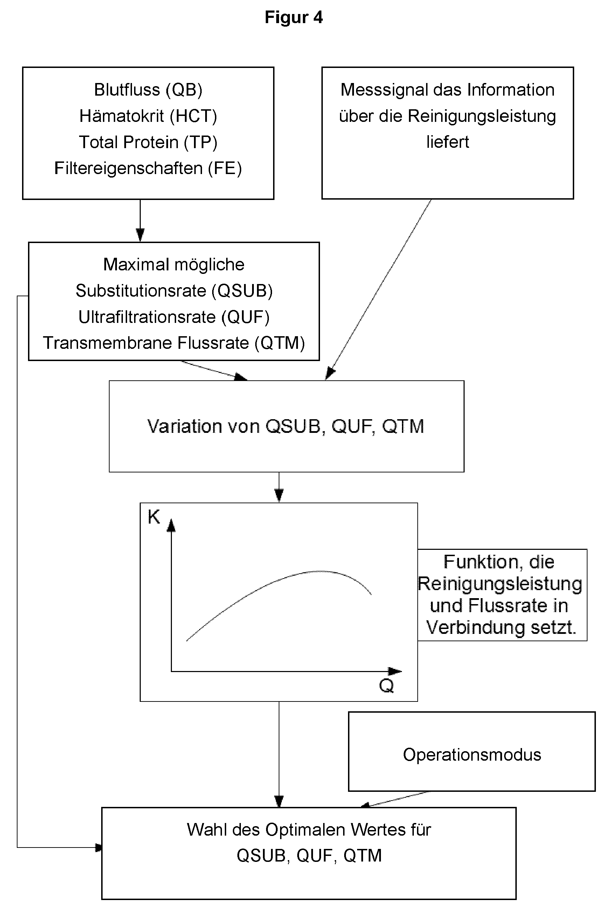

Fig. 4 zeigt eine Übersicht über die Faktoren, die beim erfindungsgemäßen Verfahren zur Einstellung der Flussrate der Substitutionslösung eine Rolle spielen. Die mittelmolekulare Reinigungsleistung (K) ist erfindungsgemäß von der Flussrate der Substitutionslösung (Q) abhängig. Dabei führt eine maximale Flussrate der Substitutionslösung (QSUB) aber nicht zu einer maximalen mittelmolekularen Reinigungsleistung (QK). Je nach dem zu erreichenden Ziel der Dialyse gibt es verschiedene Punkte bzw. Bereiche, in denen die optimale Einstellung der Flussrate der Substitutionslösung (Q) zu der optimalen, der effizientesten, der ökonomischsten oder der anderweitig definierten mittelmolekularen Reinigungsleistung führt. Die Flussrate der Substitutionslösung ist dabei aber nicht beliebig veränderbar, sondern ist auch von der Ultrafiltrationsrate (UF) und der Transmembranen Flussrate (QTM) abhängig. Die Anpassung erfolgt innerhalb der für den Patienten maximal möglichen **Substitutionsrate** (QSUB), Ultrafiltrationsrate (QUF) und transmembranen Flussrate (QTM), um die gewünschte mittelmolekulare Reinigungsleistung (K) zu erreichen. Die maximal möglichen Raten wiederum können von Patient zu Patient unterschiedlich sein und werden unter anderem beeinflusst durch den Blutfluss (QB), den Hämatokrit (HCT), dem Total Protein (TP) und/oder Filtereigenschaften (FE). Die Anpassung der mittelmolekularen Reinigungsleistung an das gewünschte Ziel erfolgt über die Variation der Flussrate der Substitutionslösung (Q), der Ultrafiltrationsrate (UF) und/oder der Transmembranen Flussrate (QTM) bzw. über andere Faktoren, die auf eine der drei Faktoren Q, UF oder TM einen Einfluss haben. Zu den anderen Faktoren gehören z.B. der Transmembrandruck, dialysatseitige Drücke, erhöhter Druckabfall über den Filter als Anzeichen für Hämokonzentration, Messung von Eingangs- und/oder Ausgangshämatokrit am Filter sowie Messung der Flussraten im Schlauch vor dem Filter und/oder dahinter. Die Messung der mittelmolekularen Substanzen auf der Dialysatseite gibt dann Aufschluss darüber, welche Flussrate der Substitutionslösung zu dem gewünschten Ergebnis führt.

In den erfindungsgemäßen Ausführungsformen kann an einer zentralen Recheneinheit ein Operationsmodus vom Anwender vorgegeben werden. Je nachdem, welche mittelmolekulare Reinigungsleistung für den jeweiligen Patienten gewünscht ist, kann der Operationsmodus z.B. auf eine optimale, effiziente oder ökonomische mittelmolekulare Reinigungsleistung gerichtet sein. Die zentrale Recheneinheit gleicht nun die aktuell eingehenden Messwerte (in Form der Flussraten und mittelmolekularen Absorbanz) mit den für diesen Steuerungsmodus hinterlegten Werten ab und passt dann nach einem der erfindungsgemäßen Verfahren die Flussrate der Substitutionslösung an. Die zentrale Recheneinheit kann jedes Gerät sein, das über eine CPU, eine Anzeigevorrichtung und eine Eingabevorrichtung verfügt, beispielsweise ein Computer. Die zentrale Recheneinheit kann an dem Dialysegerät selbst angebracht sein oder unabhängig davon betrieben werden. Gleiches gilt für eine Anzeigevorrichtung für die ermittelten Messwerte. Deren Anzeige kann über die Anzeigevorrichtung an der zentralen Recheneinheit oder über eine eigene Anzeigevorrichtung erfolgen. Die Übertragung der Messwerte kann über alle im Stand der Technik bekannten Vorrichtungen erfolgen, beispielsweise über eine Kabelverbindung oder eine WLAN-Verbindung.

Der Verlauf der Reinigungsleistung kann mit einem auf der zentralen Recheneinheit hinterlegten modellhaften Verlauf verglichen werden und eine entsprechende Korrekturmaßnahme kann vorgeschlagen oder vorgenommen werden.

Für jeden Patienten können die bei seinen Dialysesitzungen ermittelten Messwerte und Flussraten auf einem Datenträger festgehalten werden und zur Wiederverwendung zur Verfügung gestellt werden.

In weiteren Ausführungsformen können auch die baubedingten Spezifikationen des Dialysegerätes zur Optimierung des Blutreinigungsprozesses herangezogen werden. Hierbei können auch die patientenspezifischen Daten und die Spezifikationen des Dialysegerätes kombiniert werden.

Die erhobenen Daten können zur Erstellung von Flussprofilen verwendet werden, die wiederum dem Anwender vorgeschlagen werden können.

Auf der Anzeigevorrichtung kann der laufende Optimierungsprozess dem Anwender angezeigt werden. Diese Daten können auf einem Datenträger gespeichert werden. In gleicher Weise können die eingesparte Menge an Substitutionslösung und/oder die gesteigerte Reinigungsleistung bei mittelmolekularen Substanzen angezeigt werden. Somit sind die erfindungsgemäßen Verfahren dazu geeignet, die eingesparte Menge an Substitutionslösung oder die gesteigerte Reinigungsleistung bei mittelmolekularen Substanzen zu ermitteln.

Der Begriff "Operationsmodus", wie hierin verwendet, beschreibt einen voreingestellten Modus, in dem die notwendigen Parameter für die angestrebte mittelmolekulare Reinigungsleistung hinterlegt sind. Aufgrund dieser Parameter wird die für die angestrebte mittelmolekulare Reinigungsleistung notwendige Flussrate der Substitutionslösung bestimmt und dann ausgegeben und/oder automatisch eingestellt. Es sei angemerkt, dass auch weitere Operationsmodi anhand der erfindungsgemäßen Verfahren bestimmt und gegebenenfalls miteinander kombiniert werden können. Die Grenzziehung bezüglich der Werte und der Benennung der einzelnen Operationsmodi kann daher variieren.

Bei dem Signal kann es sich um ein optisches Messsignal ausgewählt aus der Gruppe bestehend aus UV-Adsorbanz, Absorbanz im sichtbaren Wellenlängenbereich, IR-Signal, zu einer Absorbanzmessung weiterverarbeitetes IR-Signal und Fluoreszenzsignal oder um ein akustisches Messsignal ausgewählt aus der Gruppe bestehend aus Änderungen der akustischer Intensität oder der akustischen Frequenz handeln.

Zur Messung der Absorbanz der mindestens einen mittelmolekularen Substanz werden bevorzugt photospektroskopische Methoden verwendet. Hierbei fällt mindestens ein Messstrahl senkrecht zur Flussrichtung der Dialyselösung ein. Bevorzugt befindet sich die Messeinheit im Dialysatausfluss. Hierzu gibt es mehrere Methoden:

a) Ein Infrarot- (IR-) Messstrahl wird bereits bei vielen modernen Dialysemaschinen eingesetzt. Er dient zur Detektion von sog. Blutlecks in der semipermeablen Membran des Dialysemoduls. Wenn hier ein Leck auftritt, gelangt Blut ungefiltert in die Dialyselösung und wird dadurch der Rückführung in den Körper des Patienten entzogen. Je nach Ausmaß des Lecks kann dies lebensgefährlich für einen Patienten werden. Das ausgetretene Blut kann durch eine Trübung in der Dialyselösung detektiert und ein Alarmsignal ausgelöst werden. Daraufhin kann die Dialysesitzung unter- oder abgebrochen werden. Das Problem beim Messen wässriger Lösungen sind jedoch zwei sehr breite charakteristische Banden für $H_2O$, die viele Messwerte überdecken. IR-Messungen sind daher nur für die Konzentrationsmessungen von Interesse, bei der die Leitsubstanz mindestens eine charakteristische Bande außerhalb der beiden $H_2O$-Banden aufweist.

b) Polarisiertes Licht kann auch nur bei Leitsubstanzen verwendet werden, die ein chirales Zentrum haben und falls sie ein solches haben, nicht als Racemat vorliegen. Bei Leitsubstanzen, die diese Bedingung erfüllen, würde sich die Konzentration c wie folgt berechnen:

$$c = \alpha \, / \, (\alpha_t \cdot d)$$

($\alpha$ = gemessener Drehwinkel; $\alpha_t$ = spezifisches Drehvermögen einer Substanz; d = Messstrecke, hier der Durchmesser des Dialysatausflusses; $\alpha$ und $\alpha_t$ sind wellenlängenspezifisch).

c) Einige der mittelmolekularen Substanzen haben charakteristische Absorptionsmaxima im Bereich des für Menschen sichtbaren Lichtes (400 - 750 nm Wellenlänge). Daher ist es möglich, daß eine Lichtquelle mit einem für diesen Bereich des sichtbaren Lichtes spezifisch regelbaren Emissionsspektrums diese spezifischen Wellenlängen senkrecht zum Dialysatfluss abgibt und die entsprechende Absorbanz gemessen wird.

d) Besonders bevorzugt wird daher die Verwendung mindestens einer UV-Absorbanz-Messeinheit. Hierbei wird ein UV-Lichtstrahl durch ein Segment der zu messenden Lösung geleitet und von einem Empfänger gemessen. Aus diesem Messwert läßt sich die Extinktion, oder als Kehrwert die Transmission, bei einer bestimmten Wellenlänge im UV-Bereich (1 - 400 nm Wellenlänge) berechnen. Die Extinktion ist für einen großen Konzentrationsbereich bei den meisten Substanzen linear zur Konzentration. Diese wird allgemein durch das Lambert-Beersche-Gesetz beschrieben:

$$E_\lambda = \varepsilon_\lambda \cdot c \cdot d$$

Die Extinktion $E_\lambda$, ist das Produkt aus dem stoffspezifischen molaren Extinktionskoeffizienten $\varepsilon_\lambda$, der Stoffkonzentration und der Wegstrecke des gemessenen Lichtes d.
Da $\varepsilon_\lambda$, und d für eine Apparatur festgelegt sind, ist $E_\lambda$, ein direktes Maß für die zu messende Stoffkonzentration.
Für typische Mittelmoleküle liegt die maximale Absorbanz bei

| Molekül | Absorbanzmaximum in nm |
|---|---|
| beta2 Mikroglobulin | 280 nm |
| Cystatin C | 280 nm |
| Retinol Binding Protein | 280 nm |
| Complement-Faktor D | 280 nm |

Man sieht hier, dass die Absorbanz im UV Bereich liegt und für die hier angegebenen exemplarischen Mittelmoleküle gleich ist. Dies liegt daran, dass die für die Absorbanz entscheidende Aminosäure das Tryptophan ist. Diese hat ihr Absorptionsmaximum bei der angegebenen Wellenlänge.
Phenol liegt bei ca. 255 nm und Tyrosin bei 275 nm.
Die UV-Messung ist stark temperaturabhängig, da die Temperatur direkt in den Extinktionskoeffizienten $\varepsilon_\lambda$, eingeht. Daher werden in bevorzugten Ausführungsformen Vorrichtungen getroffen, dass die Temperatur zumindest im Dialysatausfluss konstant gehalten wird, um eine Vergleichbarkeit der Messwerte zu gewährleisten. Bei der Bauweise der meisten modernen Dialysemaschinen ist der Dialysatausfluss genügend weit von dem Dialysator und Bereichen der Dialysemaschine, die sich erwärmen, entfernt. Je nach Bauweise können auch Platzgründe für eine Anbringung im Dialysatausfluss sprechen.
Für die UV-Absorbanzmessung können handelsübliche Sensoren verwendet werden. In bevorzugten Modellen fließt der Flüssigkeitsstrom durch ein transparentes Ausflussteil, beispielsweise einen Schlauch, der durch eine zentrale Aussparung am UV-Sensor geführt wird. In anderen Worten, der UV-Sensor umgibt das Ausflussteil in einem Abschnitt ringförmig. Der UV-Strahl fällt hierbei durch das zentrale Segment des Ausflussteiles in seiner ganzen Breite. Dies führt zu einer Erhöhung der Messgenauigkeit, da Streuungs- und Beugungseffekte so reduziert werden. Bei der Auswahl des zumindest in diesem Abschnitt transparenten Wandmaterials des Ausflussteiles ist es vorteilhaft, wenn Materialien verwendet werden, bei denen nur eine geringe Lichtstreuung und Reflexion auftritt. Diese beiden Effekte wirken sich ebenfalls negativ auf die Messgenauigkeit aus. Insbesondere bei Messungen unterhalb von 200 nm Wellenlänge hat sich Quarzglas bewährt. Erfindungsgemäß kann dieses Glas auch in den Sensor eingebaut sein. Auf der Wegstrecke durch den Sensor fließt der Dialysatausfluss frei durch dieses Glasrohr. Entsprechende Anschlüsse vor und nach dem Sensor herzustellen liegt im Wissensbereich des durchschnittlichen Fachmanns auf diesem Gebiet. Die Fehlergenauigkeit des Messsystems sollte bevorzugt unter 15% liegen, bevorzugter unter 10% und am meisten bevorzugt unter 5%.
Aus Schutzgründen sollten, wenn aufgrund der Bauweise nötig, Sichtblenden um den Messbereich des UV-Sensors angebracht sein, um zu verhindern, dass menschliches Gewebe, vor allem aber die Augen des Personals und/oder der Patienten, mit dem UV-Messstrahl in Berührung kommt.
e) Bei der Fluoreszenzmessung wird das eingestrahlte Licht von den Molekülen absorbiert (Absorptions- oder Anregungswellenlänge) und dann wieder mit größerer Wellenlänge emittiert. Dies ist durch den in der Literatur ausführlich beschriebenen Stokes-Shift zu erklären. Die Zwei-Photonen-Anregung kann auch verwendet werden. Hier nimmt das Molekül zwei Photonen der Anregungswellenläge auf um den angeregten Zustand zu erreichen und dann durch Emission eines energiereicheren Photons (kleinere Wellenlänge) wieder in den Grundzustand zu fallen. Hierfür sind aber sehr hohe Strahlungsintensitäten notwendig, da beide Photonen quasi gleichzeitig (innerhalb von $10^{-15}$ s) absorbiert werden müssen. Vorteil hier ist, dass zur Anregung größere Wellenlängen verwendet werden können, die eine größere Eindringtiefe haben als Photonen kleinerer Wellenlänge. Typischerweise werden Anregungslichtquelle und Fluoreszenzdetektor im 90° Winkel zu einander angeordnet, um die Fluoreszenzintensität einfach messen zu können. Es können aber auch Filter verwendet werden, die Anregung und Fluoreszenz trennen.

Das gewonnene Fluoreszenzsignal gibt Aufschluss über die Konzentration des fluoreszierenden Stoffs im Dialysat.

Die typischen Anregungswellenlängen sind die zuvor erwähnten für Tyrosin, Tryptophan und Phenol. Auf Grund der unterschiedlichen Molekülstruktur weichen die Fluoreszenzwellenlängen der einzelnen Moleküle von einander ab, auch wenn die aktiven Aminosäuren gleich sind.

Der Vorteil der Fluoreszenzmessung liegt darin, dass durch die leicht unterschiedliche Emission zwischen einzelnen Molekülen unterschieden werden könnte, sollte dies nötig sein.

| Molekül | Fluoreszenzmaximum in nm |
|---|---|
| beta2 Mikroglobulin | 338 nm |
| Retinol Binding Protein | 335 nm |
| Complement-Faktor D | 310 nm |

f) Zur akustischen Messung wird eine Quelle und ein Empfänger verwendet. Gemessen wird wie stark Frequenz und Amplitude durch das Dialysat und die enthaltenen mittelmolekularen Substanzen verändert wird. Die Amplitude wird geschwächt werden, die Frequenz durch den Doppler-Effekt verschoben. Die Art der Verschiebung hängt von der Teilchenart und -anzahl ab.

Die Abtastrate (sampling rate) für das jeweilige Signal, das durch mindestens eine mittelmolekulare Substanz erzeugt wird, kann entweder vom Hersteller voreingestellt sein oder von den Anwendern eingegeben werden. Erfindungsgemäß liegen die Abtast- und Regelintervalle zwischen 0,5 Sekunden und 30 Minuten, bevorzugt zwischen 1 Sekunde und 10 Minuten, bevorzugter zwischen 2 Sekunden und 5 Minuten und am meisten bevorzugt zwischen 10 Sekunden und 1 Minute.

In einigen Ausführungsformen können sich jedoch die Messintervalle während einer Dialysebehandlung verlängern, entweder gemäß einem vorgegebenen Algorithmus oder nach individuellen Einstellungen. Hintergrund hierfür ist, dass sich die Behandlungsparameter erfahrungsgemäß bei Beginn einer Dialysebehandlung stärker ändern als gegen Ende, an dem sich meist ein "quasi steady state" einstellt.

In weiteren Ausführungsformen werden zunächst mindestens zwei Messpunkte aufgenommen. Diese werden zur Charakterisierung der Reinigungsleistung herangezogen. Ein weiterer Kurvenverlauf wird interpoliert. Hierbei können die Messpunkte mit einer konstanten Frequenz aufgenommen werden oder die Messabstände können auf den erwarteten Verlauf hin optimiert werden. Die erfindungsgemäßen Verfahren können zudem ermöglichen, dass der Verlauf der Reinigungsleistung durch die Aufnahme von einzelnen Messpunkten definiert und eine Kurve interpoliert wird, wobei die Messpunkte mit einer konstanten Frequenz oder auf den erwarteten Verlauf optimiert aufgenommen werden. Dadurch können die erfindungsgemäßen Verfahren die erwartete Reinigungsleistung sehr genau abschätzen.

Die Konzentrationsmessung der entfernten Substanz oder Substanzen kann an allen Stellen in dem Dialysekompartiment vorgenommen werden. Es wird aber bevorzugt, dass die Messung an einer Stelle vorgenommen wird, an der die maximale Konzentration der zu entfernenden Substanz in der Dialyselösung vorliegt. Dies dient dazu, die zuverlässigsten Messwerte der jeweiligen Konzentration erhalten zu können. Dies ist für gewöhnlich im Ausflussteil auf der Dialyseseite der Fall. Insbesondere bevorzugt ist die Messung am Eingang des Ausflussteiles, da beim Durchlaufen der Flüssigkeit durch das Ausflussteil die Konzentration nicht mehr erhöht wird, aber im weiteren Verlauf bereits Diffusionseffekte innerhalb des Ausflussteiles auftreten können, die die Messung beeinflussen können.

In einer bevorzugten Ausführungsform wird das Signal von Substanzen erzeugt, die ein Molekulargewicht zwischen 500 und 50000 Dalton besitzen (mittelmolekulare Substanzen).

Bei der Anbindung des Ausflussteiles an den Dialysefilter hat es sich als vorteilhaft erwiesen, dass keine harten Kanten oder abrupten Verengungen des Flussweges auftreten. Es soll ein möglichst laminares Strömungsprofil, wie es für gewöhnlich in dem Dialysefilter vorliegt, erhalten bleiben. Andernfalls könnten an Kanten und Engstellungen Turbulenzen auftreten, was an diesen Stellen zu einer Ungleichverteilung der transportierten Stoffe und damit potentiell zu nicht exakten Messwerten führen kann. Da diese Turbulenzen abhängig von der regulierbaren Strömungsgeschwindigkeit sind und davon auszugehen ist, dass diese Konzentrationsänderungen nicht-linear sein können, können Turbulenzen eine Fehlerquelle darstellen. Handelsübliche Dialyseschläuche erfüllen dieses Kriterium.

Unter Schritt b) "Messung eines Signals, das durch mindestens eine mittelmolekulare Substanz im Dialysatfluss der Blutbehandlungseinheit erzeugt wird" soll verstanden werden, dass mittels der Messvorrichtung z.B. die UV-Absorbanz, die Absorbanz im sichtbaren Wellenlängenbereich, ein IR-Signal, ein Fluoreszenzsignal, eine akustische Intensität und/oder eine akustische Frequenz gemessen wird, welches durch eine Spezies eine mittelmolekularen Substanz oder durch mehrere verschiedene mittelmolekulare Substanzen im Dialysatfluss der Blutbehandlungseinheit erzeugt wird. In der Regel wird es sich um ein überlagertes Signal von verschiedenen mittelmolekularen Substanzen handeln. Erfindungsgemäß kann dieses Signal bei einer bestimmten Wellenlänge oder Intensität gemessen werden. Es können aber auch mehrere Signale gleichzeitig oder zeitversetzt jeweils bei verschiedenen Wellenlängen oder Intensitäten gemessen werden oder verschiedenartige Signale wie z.B. die UV-Absorbanz zusammen mit der IR-Absorbanz gemessen werden. Die Messung des Signals erfolgt natürlich bei derselben Wellenlänge oder Intensität. Im Falle der Erfassung mehrerer Signale, werden die mehreren Signale natürlich jeweils auch wieder bei derselben Wellenlänge oder Intensität gemessen.

Der Begriff "Clearance" und "Reinigungsleistung", wie hierin verwendet, bezeichnet das Entfernen mindestens einer bestimmten exo- oder endogenen Substanz aus dem Blut des Patienten. Beide Begriffe können synonym verwendet werden. Der Begriff "Clearance" oder synonym "Reinigungsleistung" steht für die Konzentration einer exo- oder endogenen Substanz, welche dem Blut durch diffusiven Stofftransport und konvektiven Stofftransport entzogen wird. Die "Clearance" oder synonym die "Reinigungsleistung" hat die Einheit Volumen pro Zeit [vorzugsweise ml/min].

Die vorliegende Erfindung betrifft die Entfernung von mittelmolekularen Substanzen, so dass sich der Begriff "mittelmolekulare Clearance" oder synonym "mittelmolekulare Reinigungsleistung" oder synonym "Reinigungsleistung für mittelmolekulare Substanzen" (Abkürzung K) auf die Entfernung von mittelmolekularen Substanzen aus dem Blut bezieht. Die "mittelmolekulare Clearance" oder synonym "mittelmolekulare Reinigungsleistung" oder synonym "Reinigungsleistung für mittelmolekulare Substanzen" abgekürzt "K" kann durch folgende Formel berechnet werden:

$$K = Q_b \left( 1 - \frac{c_{aus}}{c_{ein}} \right) Q_{UF} \frac{c_{aus}}{c_{ein}}$$

nach Krieter et al., 2004
worin

$Q_b$ die Blutflussrate ist, und
$Q_{UF}$ die Ultrafiltrationsrate bezeichnet.

Der erste Teil der Formel beschreibt die Clearance für den diffusiven Stofftransport und den Anteil an konvektiver Clearance, der durch Substitution erzeugt wird. Der zweite Teil der Formel beschreibt den Anteil, der durch konvektiven Stofftransport erzeugt wird, der aber nicht wieder substituiert wird, also die zusätzliche Clearance erzeugt durch den Flüssigkeitsentzug.

Das erfindungsgemäße Verfahren kann grundsätzlich an allen Säugetieren durchgeführt werden. Bevorzugt wird jedoch eine Verwendung bei der Behandlung menschlicher Patienten.

Unter dem Oberbegriff Blutbehandlungseinheit werden alle Vorrichtungen verstanden, die zur Reinigung und/oder Behandlung von Blut eingesetzt werden können. Die am häufigsten eingesetzten Verfahren sind die Hämodialyse, Peritonealdialyse, Hämoperfusion, Hämofiltration und Hämodiafiltration.

Der Begriff Dialyse bezieht sich auf Reinigungsverfahren, bei denen zwei Flüssigkeitsströme durch eine permeable Membran voneinander getrennt sind, die aber den gewünschten Stoffaustausch ermöglicht. Der eine Flüssigkeitsstrom, im vorliegenden Fall Blut, führt die zu entfernenden Stoffe mit sich, während der andere Strom mit der Dialyselösung diese Stoffe aufnehmen soll.

Der Begriff Ultrafiltration bezieht sich hierbei auf die Filtration über eine Membran mit einem Druck von 0 - 1 bar, bei der Teilchen größer als ca. 0,01 $\mu$m zurückgehalten werden sollen.

Der Begriff "Kontinuierliche Messung", wie hierin verwendet, bezieht sich auf den erfindungsgemäßen Vorgang der Ermittlung des Signals bei unterschiedlichen Flussraten der Substitutionslösung und beschreibt die Messung des Signals der mindestens einen mittelmolekularen Substanz bei mindestens zwei unterschiedlichen Flussraten der Substitutionslösung. Bevorzugt erfolgt die Messung, bis das erfindungsgemäße Verfahren abgeschlossen ist und die gewünschte Flussrate der Substitutionslösung eingestellt wurde. Mit "Kontinuierliche Messung" ist nicht zwingend gemeint, dass das erfindungsgemäße Verfahren über die gesamte Dauer der Dialyse durchgeführt werden muss. Es ist möglich, dass die Messung in zeitlich festgelegten Intervallen wiederholt wird, um die gewünschte Flussrate der Substitutionslösung über die Dauer der Dialyse den Systemveränderungen anzupassen. Dieser Vorgang kann auch manuell vom Personal oder dem Patienten ausgelöst werden.

Bei den bekannten Hämo(dia)filtrationsvorrichtungen wird die Substitutionslösung dem extrakorporalen Blutkreislauf von dem Flüssigkeitssystem der Maschine über eine Substitutionszuführleitung zugeführt. Bei der Prädilution wird die Substitutionslösung dem Blut vor dem Durchfließen des Dialysators oder Filters zugeführt, während bei der Postdilution die Substitutionslösung dem Blut nach dem Durchfließen des Dialysators oder Filters zugeführt wird. Die Substitutionsleitung weist beispielsweise einen Konnektor auf, mit dem sie entweder an die venöse (nach dem Dialysator gelegene) oder an die arterielle (vor dem Dialysator gelegene) Blutleitung angeschlossen werden kann. Zum Unterbrechen der Flüssigkeitszufuhr ist an der Substitutionsleitung eine Klemme oder dgl. vorgesehen. Erfindungsgemäß spielt es keine Rolle, an welcher Stelle des Kreislaufs die Substitutionszufuhr erfolgt, da es sich hierbei nur um eine sekundäre Größe handelt, die der Reinigungsleistung in Form der mittelmolekularen Reinigungsleistung angepasst wird.

Die vorliegende Erfindung bezieht sich ebenfalls auf eine Vorrichtung in verschiedenen Ausführungsformen, die die zuvor beschriebenen Verfahren ausführen können.

Somit bezieht sich die vorliegende Erfindung auch auf eine Blutbehandlungseinheit, an der die Reinigungsleistung für mittelmolekulare Substanzen über die Messung eines Absorbanzsignals eingestellt werden kann, die die folgenden Bauteile umfasst:

- einen Tangentialflussfilter;
- eine Messvorrichtung zur kontinuierlichen Messung des besagten Signals mindestens einer mittelmolekularen Substanz im Dialysatfluss der Blutbehandlungseinheit, wobei die besagte Messvorrichtung die UV-Absorbanz, die Absorbanz im sichtbaren Wellenlängenbereich, ein IR-Signal, ein Fluoreszenzsignal, die akustische Intensität und/oder die akustische Frequenz messen kann;
- Mittel zur Variation der Flussrate der Substitutionslösung während der kontinuierlichen Messung;
- Mittel zur Weiterleitung der Flussraten der Substitutionslösung und der korrespondierenden Messwerte der Absorbanz der mindestens einen mittelmolekularen Substanz im Dialysatfluss an eine zentrale Recheneinheit;
- eine zentrale Recheneinheit;
- Mittel zur Korrelation der Flussraten der Substitutionslösung mit der Absorbanz der mindestens einen mittelmolekularen Substanz auf der zentralen Recheneinheit, wobei die Korrelation beinhaltet:

   Bestimmen der Reinigungsleistung für die mindestens eine mittelmolekulare Substanz (K0) vor Änderung des Substituatflusses;
   Bestimmen der Reinigungsleistung für die mindestens eine mittelmolekulare Substanz (K1) nach Änderung des Substituatflusses;
   Bestimmen der Änderung des Substituatflusses in ml/min ($\Delta$S);
   Berechnung der Änderung der Reinigungsleistung (P) für die mindestens eine mittelmolekulare Substanz in Abhängigkeit von der Änderung des Substituatflusses ($\Delta$**S**) gemäß:

$$\Delta K(0,1) = K1-K0$$

$$P = (\Delta K(0,1)/\Delta S)*100\%$$

   wobei im Operationsmodus Optimum bestimmt wird, ob eine Erhöhung des Substituatflusses zumindest noch eine Steigerung von $10\% < P < 40\%$ bewirkt,
   wobei im Operationsmodus Efficiency bestimmt wird, ob eine Erhöhung des Substituatflusses zumindest noch eine Steigerung von $40 \leq P < 70\%$ bewirkt,
   wobei im Operationsmodus Economy bestimmt wird, ob eine Erhöhung des Substituatflusses zumindest noch eine Steigerung von $P \geq 70\%$ bewirkt; und

- eine Anzeigevorrichtung für das Ergebnis der Korrelation der Flussraten der Substitutionslösung mit der Absorbanz.

Die hierin beschriebenen Blutbehandlungseinheiten sind zur Ausführung der hierin beschriebenen Verfahren konzipiert, so dass sämtliche Ausführungen zu den erfindungsgemäßen Verfahren natürlich auch für die erfindungsgemäßen Blutbehandlungseinheiten gelten, welche die erfindungsgemäßen Verfahren ausführen.

In bevorzugten Ausführungsformen kann diese Vorrichtung zusätzlich Mittel zur automatischen Regulation der Flussrate der Substitutionslösung anhand des bei der Korrelation ermittelten Ergebnisses umfassen.

In weiteren bevorzugten Ausführungsformen kann diese Vorrichtung eine Auswahlmöglichkeit auf der zentralen Recheneinheit umfassen, über die der Anwender einen Operationsmodus ausgewählt aus der Gruppe Eco Modus (Economy Modus), Efficiency Modus und Optimum Modus vorwählen kann.

Eine solche Auswahlmöglichkeit kann beispielsweise ein vom Anwender zu bedienender Schalter oder ein Eingabefeld auf einer Benutzeroberfläche der Anzeige der zentralen Recheneinheit sein.

**Figuren**

Fig. 1: Abhängigkeit der Clearance von der eingestellten Substitutionsrate. Aufgenommen wurden diese Daten bei einem Blutfluss von 300 ml/min.

Fig. 2: Kurvenverlauf der theoretischen Darstellung einer Effizienzkurve

Fig. 3: Theoretische prozentuale mittelmolekulare Reinigungsleistung (Clearance) über die Zeit bei konstanter Konzentration auf der Blutseite.

a) Kurve mit konstanter Substitutionsrate (Stand der Technik) [untere Kurve: Standardverlauf]
b) Kurve mit einer erfindungsgemäß geregelten Substitutionsrate [mittlere Kurve: Substituatsteuerung]
c) Kurve mit einer erfindungsgemäß geregelten Substitutionsrate wie in b), jedoch mit intermittierenden Spülungen [obere Kurve: Substituatsteuerung mit stündlichem Spülvorgang]

Man erkennt, dass die Kurve a) den stärksten Abfall in der Reinigungsleistung aufweist. Durch die beschriebene automatische Anpassung des Substitutionsflusses wird die Reinigungsleistung auf einem höheren Niveau gehalten und die Dialysequalität wird verbessert (vgl b). Ergänzt man diese noch durch Spülvorgänge um die Filterqualität möglichst hoch zu halten, erhält man die oberste Kurve (c). Hier ist dargestellt wie durch die Spülvorgänge die Filterqualität verbessert wird und so die Reinigungsleistung sprunghaft steigt, dann aber wieder bis zum nächsten Vorgang fällt.

Fig. 4: Flussdiagramm des erfindungsgemäßen Verfahrens Dargestellt sind die Eingangsparameter Blutfluss, Hämatokrit, total Protein (Gesamtprotein) und die Filtereigenschaften (Oberfläche, Permeabiliät, Faserinnendurchmesser, Faserlänge und Faseranzahl) auf den maximal einstellbaren transmembranen Fluss, der sich aus Substitutionsrate und Ultrafiltrationsrate zusammensetzt. Da die Ultrafiltrationsrate ein medizinisch kritischer Parameter ist, der durch den Arzt einzustellen ist, wird die der Beschreibung der Erfindung immer die Substitutionsrate als zu regelnder Parameter betrachtet, es wäre natürlich auch möglich die Ultrafiltrationsrate mit einzubeziehen. Hat man den maximal möglichen Fluss bestimmt, kann die Flussrate im Rahmen des erlaubten Bereichs variiert werden. Verfügt man nun über ein Messsignal, das Informationen über die aktuelle Reinigungsleistung liefert, so kann dieses in Zusammenhang mit der transmembranen Flussrate gebracht werden.
Durch Auswertung dieses Zusammenhangs (dieser Funktion) unter Berücksichtigung des Operationsmodus kann nun der Arbeitspunkt für die Substitutionsrate bestimmt werden. Diese kann noch einmal mit dem zuvor bestimmten möglichen Maximalwert verglichen werden um diesen nicht zu überschreiten.

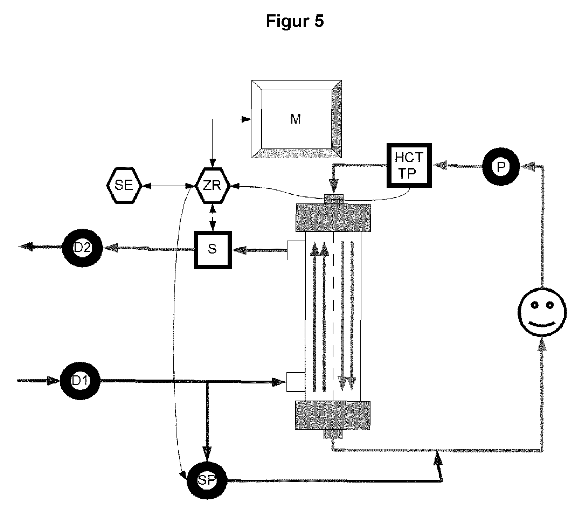

Fig. 5: Dargestellt ist eine Umsetzungsform einer zuvor beschriebenen Steuereinheit. Blut wird in einem extrakorporalen Kreislauf mittels einer Pumpe (P) zirkuliert. Hierbei fließt es durch einen Tangentialflussfilter, den Dialysator, in dem der Reinigungsprozess stattfindet. Hinter dem Dialysator befindet sich eine Anschlussmöglichkeit über die im Fall der Postdilution eine physiologische Flüssigkeit zugegeben wird um die im Filter entzogene Flüssigkeitsmenge auszugleichen. Im Blutkreislauf befinden sich Messmittel, die Hämatokrit und / oder Gesamtprotein (HCT TP) ermitteln können.
Dieses Beispiel bezieht sich auf eine online HDF Therapie, bei der aus dem Dialysatkreislauf angetrieben durch die Pumpen (D1 und D2) frische Dialysierlösung mit der Substituatpumpe (SP) abgezweigt wird und dem Blut zugeführt wird, nachdem es den Filter durchlaufen hat. Dargestellt ist also ein Betrieb im Postdilutions-Modus. Die SP wird durch eine Zentrale Recheneinheit (ZR) gesteuert, die anhand eines durch den Sensor (S) zur Überwachung der mittelmolekularen Reinigungsleistung generierten Signals und dessen Abhängigkeit von der Substitutionsrate einen bestimmten Wert für die SP vorgibt. Auf einer Speichereinheit (SE) sind bekannte Behandlungsverläufe hinterlegt, die die ZR zur Einstellung der SE heranziehen kann.
Die durch die ZR eingestellte Substitutionsrate, das daraus errechnete Substitutionsvolumen, die erzielte Reinigungsleistung sowie eine Vorhersage der Reinigungsleistung für das Therapieende können auf dem Monitor (M) angezeigt werden. Der Anwender hat über den Monitor auch die Möglichkeit den Operationsmodus zu wählen, der in die Ermittlung der Substitutionsrate genauso eingeht wie der Hämatokrit, Gesamtprotein, Blutfluss und die Filtereigenschaften.

**Beispiele**

Beispiel 1:

Rinderblut (Hämotakrit Htc = 32%; hoher Heparinisierung; 5000 IU; c(beta2-Mikroglobulin) = 85 mg/l) wurde durch eine Dialysemaschine des Bautyps Dialog⁺ geleitet, die mit der HDF-online Option ausgestattet ist. Die Reinigungsleistung wurde über eine UV-Absorbanzmessung bei 280 nm im Dialysat gemessen. Das Rinderblut war durch Durchleitung durch eine Heizschlange auf 37 ± 1°C temperiert worden. Der Blutfluss betrug 300 ml/min, Dialysatfluss 600 ml/min.

Das Substitutionsvolumen geht unmittelbar aus der Substituatflussrate hervor. Der erste Messpunkt wurde bei einem Substituatfluss von 0 ml/min aufgenommen. Für die weiteren Messpunkte wurde der Substituatfluss jeweils um ein Inkrement von 20 ml/min bis auf einen Endwert von 120 ml/min erhöht. Vor dem Ablesen des jeweiligen Messwertes wurde jeweils mindestens 2 min abgewartet, um abzuwarten, daß sich die Reinigungsleistung stabilisiert hatte.

Der Messwert der UV-Absorbanz wurde durch eine Eichkurve in eine Konzentration für beta2-Mikroglobulin umgerechnet.

Das Experiment wurde mehrmals mit einer jeweils neuen und vergleichbaren Probe Rinderblut wiederholt (n = 3). Die Messwerte sind als Mittelwert ± Standardabweichung angegeben.

Der Zusammenhang zwischen erzeugter Clearance (Reinigungsleistung) und Substituatfluss verläuft im Anfangsbereich der Kurve fast linear und fing ab 100 ml/min Substitutionsrate an in ein Plateau überzugehen. Bei dieser Versuchsreihe wurde keine Abnahme der Reinigungsleistung bei einer weiteren Erhöhung der Geschwindigkeit des Substituatflusses beobachtet.

Beispiel 2:

Nach dem gleichen Prinzip wie in Beispiel 1 wurden Fluoreszenzmessungen des Dialysats durchgeführt.

Nun wurde allerdings keine Absorbanz gemessen, sondern die durch das beta2-Mikroglobulin verursachte Fluoreszenz bei 338 nm.

Es wurden die Fluoreszenzdaten der Messung erneut mit einer Eichkurve verglichen, die den Fluoreszenzintensitäten einen Konzentrationswert zuordnet.

Es trat das Verhalten wie bei der UV-Absorbanz-Messung auf. Auf einen quasi linearen Verlauf bis hin zu einer Substitutionsrate von 100 ml/min folgt ein deutliches Abflachen der Kurve.

Beispiel 3:

Das Versuchsprotokoll ist das gleiche gewesen wie in den Beispielen zuvor beschrieben, allerdings wurde diesmal polarisiertes Licht eingestrahlt und die Drehung der Polarisationsrichtung gemessen. Als Markermolekül wurde eine optisch aktive Substanz verwendet, die einen Drehwinkel von 5° erzeugt und eine Größe von ca. 15 kDa aufweist.

Der Analysator des Versuchsaufbaus wurde um 5° gegen die Eingangspolarisationsrichtung gedreht und die Intensität hinter dem Analysator gemessen.

Zunächst wurde das Intensitätssignal geeicht um hieraus direkt die Konzentrationen berechnen zu können, anschließend wurde der Versuch in Analogie zu den vorherigen durchgeführt.

Auch hier wurde wieder der lineare Anstieg im Anfangsbereich der Kurve gemessen, allerdings beginnt flacht die Kurve nun schon bei einer Substitutionsrate von 80 ml/min ab.

**Patentansprüche**

**1.** Verfahren zur Bestimmung einer Flussrate einer Substitutionslösung, bei der eine Reinigungsleistung für mittelmolekulare Substanzen eines Tangentialflussfilters einer Blutbehandlungseinheit erreicht wird, umfassend die folgenden Schritte:

a) Auswahl des vorher festgelegten Bereichs in Form eines Operationsmodus ausgewählt aus Optimum, Efficiency oder Economy;
b) Messung eines Signals, das durch mindestens eine mittelmolekulare Substanz im Dialysatfluss der Blutbehandlungseinheit erzeugt wird;
c) Variation der Flussrate der Substitutionslösung während der Messung nach b);
d) Weiterleitung der Flussraten der Substitutionslösung und der korrespondierenden Messwerte des Signals der mindestens einen mittelmolekularen Substanz im Dialysatfluss an eine zentrale Recheneinheit;

e) Korrelation der Flussraten der Substitutionslösung mit dem Signal der mindestens einen mittelmolekularen Substanz, wobei die Korrelation beinhaltet:

Bestimmen der Reinigungsleistung für die mindestens eine mittelmolekulare Substanz (K0) vor Änderung des Substituatflusses;
Bestimmen der Reinigungsleistung für die mindestens eine mittelmolekulare Substanz (K1) nach Änderung des Substituatflusses;
Bestimmen der Änderung des Substituatflusses in ml/min ($\Delta$S);
Berechnung der Änderung der Reinigungsleistung (P) für die mindestens eine mittelmolekulare Substanz in Abhängigkeit von der Änderung des Substituatflusses ($\Delta$**S**) gemäß:

$$\Delta K(0,1) = K1 - K0$$

$$P = (\Delta K(0,1)/\Delta S) * 100\%$$

wobei im Operationsmodus Optimum bestimmt wird, ob eine Erhöhung des Substituatflusses zumindest noch eine Steigerung von $10\% < P < 40\%$ bewirkt,
wobei im Operationsmodus Efficiency bestimmt wird, ob eine Erhöhung des Substituatflusses zumindest noch eine Steigerung von $40 \leq P < 70\%$ bewirkt,
wobei im Operationsmodus Economy bestimmt wird, ob eine Erhöhung des Substituatflusses zumindest noch eine Steigerung von $P \geq 70\%$ bewirkt,

f) Ausgabe der aus e) ermittelten Werte entsprechend dem für den Operationsmodus festgelegten Bereich.

2. Verfahren gemäß Anspruch 1, wobei es sich bei dem besagten Signal um ein optisches Messsignal ausgewählt aus der Gruppe bestehend aus UV-Adsorbanz, Absorbanz im sichtbaren Wellenlängenbereich, IR-Signal, zu einer Absorbanzmessung weiterverarbeitetes IR-Signal, Polarisationsmessung und Fluoreszenzsignal oder um ein akustisches Messsignal ausgewählt aus der Gruppe bestehend aus Änderungen der akustischer Intensität oder der akustischen Frequenz handelt.

3. Verfahren gemäß eines der vorherigen Ansprüche, wobei ein vom Anwender selbst definierter Modus ausgewählt wird.

4. Verfahren gemäß eines der vorherigen Ansprüche, wobei in vordefinierten oder frei wählbaren Zeitintervallen der Fluss der Substitutionslösung variiert wird.

5. Verfahren gemäß Anspruch 4, wobei die vordefinierten Zeitintervalle über den Verlauf der Behandlung gleich bleiben oder wobei die vordefinierten Zeitintervalle zum Ende der Behandlung größer werden.

6. Verfahren gemäß Anspruch 5, wobei die zum Ende der Behandlung größer werdenden Zeitintervalle in Abhängigkeit der Änderungsgeschwindigkeit des Messsignals angepasst werden.

7. Verfahren gemäß eines der vorherigen Ansprüche, wobei der Verlauf der Reinigungsleistung durch die Aufnahme von einzelnen Messpunkten definiert und eine Kurve interpoliert wird, wobei die Messpunkte mit einer konstanten Frequenz oder auf den erwarteten Verlauf optimiert aufgenommen werden.

8. Verfahren gemäß eines der vorherigen Ansprüche, wobei der Verlauf der Reinigungsleistung mit einem auf der zentralen Recheneinheit hinterlegten modellhaften Verlauf verglichen und eine entsprechende Korrekturmaßnahme vorgeschlagen wird.

9. Verfahren gemäß eines der vorherigen Ansprüche, wobei für jeden Patienten die bei seinen Dialysesitzungen ermittelten Messwerte und Flussraten auf einem Datenträger festgehalten und zur Wiederverwendung zur Verfügung gestellt werden.

10. Blutbehandlungseinheit, an der die Reinigungsleistung für mittelmolekulare Substanzen des Tangentialflussfilters mittels online Messung eines Signals, das in Korrelation zur mittelmolekularen Reinigung steht, eingestellt werden kann, umfassend:

- einen Tangentialflussfilter;
- eine Messvorrichtung zur kontinuierlichen Messung des besagten Signals mindestens einer mittelmolekularen Substanz im Dialysatfluss der Blutbehandlungseinheit, wobei die besagte Messvorrichtung die UV-Absorbanz, die Absorbanz im sichtbaren Wellenlängenbereich, ein IR-Signal, ein Fluoreszenzsignal, die akustische Intensität und/oder die akustische Frequenz messen kann;
- Mittel zur Variation der Flussrate der Substitutionslösung während der kontinuierlichen Messung;
- Mittel zur Weiterleitung der Flussraten der Substitutionslösung und der korrespondierenden Messwerte der Absorbanz der mindestens einen mittelmolekularen Substanz im Dialysatfluss an eine zentrale Recheneinheit;
- eine zentrale Recheneinheit;
- Mittel zur Korrelation der Flussraten der Substitutionslösung mit der Absorbanz der mindestens einen mittelmolekularen Substanz auf der zentralen Recheneinheit, wobei die Korrelation beinhaltet:

Bestimmen der Reinigungsleistung für die mindestens eine mittelmolekulare Substanz (K0) vor Änderung des Substituatflusses;
Bestimmen der Reinigungsleistung für die mindestens eine mittelmolekulare Substanz (K1) nach Änderung des Substituatflusses;
Bestimmen der Änderung des Substituatflusses in ml/min ($\Delta S$);
Berechnung der Änderung der Reinigungsleistung (P) für die mindestens eine mittelmolekulare Substanz in Abhängigkeit von der Änderung des Substituatflusses ($\Delta\mathbf{S}$) gemäß:

$$\Delta K(0,1) = K1-K0$$

$$P= (\Delta K(0,1)/\Delta S)*100\%$$

wobei im Operationsmodus Optimum bestimmt wird, ob eine Erhöhung des Substituatflusses zumindest noch eine Steigerung von $10\% < P < 40\%$ bewirkt,
wobei im Operationsmodus Efficiency bestimmt wird, ob eine Erhöhung des Substituatflusses zumindest noch eine Steigerung von $40 \leq P < 70\%$ bewirkt,
wobei im Operationsmodus Economy bestimmt wird, ob eine Erhöhung des Substituatflusses zumindest noch eine Steigerung von $P \geq 70\%$ bewirkt;

- und eine Anzeigevorrichtung für das Ergebnis der Korrelation der Flussraten der Substitutionslösung mit der Absorbanz.

**11.** Blutbehandlungseinheit gemäß Anspruch 10, zusätzlich umfassend:

Mittel zur automatischen Regulation der Flussrate der Substitutionslösung anhand des bei der Korrelation ermittelten Ergebnisses.

**12.** Blutbehandlungseinheit gemäß Anspruch 10 oder 11, zusätzlich umfassend:

eine Auswahlmöglichkeit über die der Anwender einen Operationsmodus aus der Gruppe Economy Modus, Efficiency Modus und Optimum Modus auswählen kann oder die Option einen Operationsmodus zu definieren, wobei die Auswahlmöglichkeit ein vom Anwender zu bedienender Schalter oder ein Eingabefeld auf einer Benutzeroberfläche der Anzeige der zentralen Recheneinheit ist.

**13.** Verfahren zur Bestimmung einer Flussrate einer Substitutionslösung, bei der eine Reinigungsleistung für mittelmolekulare Substanzen eines Tangentialflussfilters einer Blutbehandlungseinheit erreicht wird, umfassend die folgenden Schritte:

a) Auswahl des vorher festgelegten Bereichs in Form eines Operationsmodus ausgewählt aus Optimum, Efficiency oder Economy;

b) Messung eines Signals, das durch mindestens eine mittelmolekulare Substanz im Dialysatfluss der Blutbehandlungseinheit erzeugt wird;

c) Variation der Flussrate der Substitutionslösung während der Messung nach b);

d) Weiterleitung der Flussraten der Substitutionslösung und der korrespondierenden Messwerte des Signals der mindestens einen mittelmolekularen Substanz im Dialysatfluss an eine zentrale Recheneinheit;

e) Korrelation der Flussraten der Substitutionslösung mit dem Signal der mindestens einen mittelmolekularen Substanz, wobei die Korrelation beinhaltet:

Bestimmen der Reinigungsleistung für die mindestens eine mittelmolekulare Substanz (K0) vor Änderung des Substituatflusses;

Bestimmen der Reinigungsleistung für die mindestens eine mittelmolekulare Substanz (K1) nach einer ersten Änderung des Substituatflusses;

Bestimmen der Reinigungsleistung für die mindestens eine mittelmolekulare Substanz (K2) nach einer zweiten Änderung des. Substituatflusses;

Berechnung der Änderung der Reinigungsleistung (P) für die mindestens eine mittelmolekulare Substanz gemäß:

$$\Delta K(0,1) = K1\text{-}K0$$

$$\Delta K(1,2) = K2\text{-}K1$$

$$P = (\Delta K(1,2) \, / \, \Delta K(0,1)) * 100\%$$

wobei im Operationsmodus Optimum bestimmt wird, ob eine Erhöhung des Substituatflusses zumindest noch eine Steigerung von P > 25% bewirkt,

wobei im Operationsmodus Efficiency bestimmt wird, ob eine Erhöhung des Substituatflusses zumindest noch eine Steigerung von P > 65% bewirkt,

wobei im Operationsmodus Economy bestimmt wird, ob eine Erhöhung des Substituatflusses zumindest noch eine Steigerung von P > 85% bewirkt, und

f) Ausgabe der aus e) ermittelten Werte entsprechend dem für den Operationsmodus festgelegten Bereich.

**Claims**

1. Method for the determination of a flow rate of a substitution solution, for which a clearance for medium-molecular substances of a tangential flow filter of a blood treatment unit is achieved, comprising the following steps:

a) Selection of the predetermined range in the form of an operating mode selected from Optimum, Efficiency or Economy;

b) Measurement of a signal, which is generated by at least one medium-molecular substance in the dialysate flow of the blood treatment unit;

c) Variation of the flow rate of the substitution solution during the measurement according to b);

d) Transmission of the flow rates of the substitution solution and the corresponding measuring values of the signal of the at least one medium-molecular substance in the dialysate flow to a central processing unit;

e) Correlation of the flow rates of the substitution solution with the signal of the at least one medium-molecular substance, wherein the correlation includes:

Determination of the clearance for the at least one medium-molecular substance (K0) before change of the substitute flow;

Determination of the clearance for the at least one medium-molecular substance (K1) after change of the substitute flow;

Determination of the change of the substitute flow in ml/min ($\Delta S$);

Calculation of the change of the clearance (P) for the at least one-medium-molecular substance in dependence of the change of the substituate flow ($\Delta$S) in accordance with:

$$\Delta K(0.1) = K1-K0$$

$$P= (\Delta K(0.1)/\Delta S)\text{*}100\%$$

wherein it is determined in the operating mode Optimum, if an increase of the substituate flow still induces at least a rise of 10% < P < 40%,
wherein it is determined in the operating mode Efficiency, if an increase of the substituate flow still induces at least a rise of $40 \leq P < 70\%$,
wherein it is determined in the operating mode Economy, if an increase of the substituate flow still induces at least a rise of $P \geq 70\%$,

f) Output of the values determined in e) according to the range determined for the operating mode.

2. Method according to claim 1, wherein said signal is an optical measuring signal selected from the group consisting of UV adsorbance, absorbance in the visible wavelength range, IR signal, IR signal processed into an absorbance measurement, polarization measurement and fluorescence signal or an acoustic measuring signal selected from the group consisting of changes of the acoustic intensity or of the acoustic frequency.

3. Method according to one of the preceding claims, wherein a mode defined by the operator himself is selected.

4. Method according to one of the preceding claims, wherein the flow of the substitution solution is varied in predefined or freely selectable time intervals.

5. Method according to claim 4, wherein the predefined time intervals remain constant over the course of the treatment or wherein the predefined time intervals are getting larger towards the end of the treatment.

6. Method according to claim 5, wherein the time intervals getting larger towards the end of the treatment are adapted in dependence on the rate of change of the measuring signal.

7. Method according to one of the preceding claims, wherein the course of the clearance is defined by the recording of single measuring points and a curve is interpolated, wherein the measuring points are recorded at a constant frequency or recorded optimized to the anticipated course.

8. Method according to one of the preceding claims, wherein the course of the clearance is compared to an exemplary course deposited on the central processing unit and a corresponding corrective measure is proposed.

9. Method according to one of the preceding claims, wherein for each patient the measuring values and flow rates determined during his dialysis sessions are retained on a data storage medium and are made available for reuse.

10. Blood treatment unit, at which the clearance for medium-molecular substances of the tangential flow filter can be adjusted using an online measurement of a signal, which correlates to the medium-molecular purification, comprising:

- a tangential flow filter;
- a measuring device for the continuous measurement of said signal of at least one medium-molecular substance in the dialysate flow of the blood treatment unit, wherein said measuring device is able to measure the UV absorbance, the absorbance in the visible wavelength range, an IR signal, a fluorescence signal, the acoustic intensity and/or the acoustic frequency;
- means for the variation of the flow rate of the substitution solution during the continuous measurement;
- means for forwarding of the flow rates of the substitution solution and of the corresponding measuring values of the absorbance of the at least one medium-molecular substance in the dialysate flow to a central processing unit;
- a central processing unit;
- means for the correlation of the flow rates of the substitution solution with the absorbance of the at least one medium-molecular substance on the central processing unit, wherein the correlation includes:

Determination of the clearance for the at least one medium-molecular substance (K0) before change of the substituate flow;
Determination of the clearance for the at least one medium-molecular substance (K1) after change of the substituate flow;
Determination of the change of the substituate flow in ml/min ($\Delta$**S**);
Calculation of the change of the clearance (P) for the at least one-medium-molecular substance in dependence of the change of the substituate flow ($\Delta$**S**) in accordance with:

$$\Delta K(0.1) = K1\text{-}K0$$

$$P= (\Delta K(0.1)/\Delta S)\text{*}100\%$$

wherein it is determined in the operating mode Optimum, if an increase of the substituate flow still induces at least a rise of 10% < P < 40%,
wherein it is determined in the operating mode Efficiency, if an increase of the substituate flow still induces at least a rise of 40 $\leq$ P < 70%,
wherein it is determined in the operating mode Economy, if an increase of the substituate flow still induces at least a rise of P $\geq$ 70%;

- and a display device for the result of the correlation of the flow rates of the substitution solutions with the absorbance.

**11.** Blood treatment unit according to claim 10, additionally comprising:

Means for the automatic regulation of the flow rate of the substitution solution on the basis of the result determined by the correlation.

**12.** Blood treatment unit according to claim 10 or 11, additionally comprising:

An option by which the operator can select an operating mode from the group Economy Mode, Efficiency Mode and Optimum Mode or the option to define an operating mode, wherein the option is a switch or input screen to be used by the user on a user interface of a display of the central processing unit.

**13.** Method for the determination of a flow rate of a substitution solution, for which a clearance for medium-molecular substances of a tangential flow filter of a blood treatment unit is achieved, comprising the following steps:

a) Selection of the predetermined range in the form of an operating mode selected from Optimum, Efficiency or Economy;
b) Measurement of a signal, which is generated by at least one medium-molecular substance in the dialysate flow of the blood treatment unit;
c) Variation of the flow rate of the substitution solution during the measurement according to b);
d) Forwarding of the flow rates of the substitution solution and the corresponding measuring values of the signal of the at least one medium-molecular substance in the dialysate flow to a central processing unit;
e) Correlation of the flow rates of the substitution solution with the signal of the at least one medium-molecular substance, wherein the correlation includes:

Determination of the clearance for the at least one medium-molecular substance (K0) before change of the substituate flow;
Determination of the clearance for the at least one medium-molecular substance (K1) after a first change of the substituate flow;
Determination of the clearance for the at least one medium-molecular substance (K2) after a second change of the substituate flow;
Calculation of the change of the clearance (P) for the at least one-medium-molecular substance in accord-

ance with:

$$\Delta K(0.1) = K1\text{-}K0$$

$$\Delta K(1.2) = K2\text{-}K1$$

$$P= (\Delta K(1.2) \,/\, \Delta K(0.1))*100\%$$

wherein it is determined in the operating mode Optimum, if an increase of the substituate flow still induces at least a rise of P > 25%,
wherein it is determined in the operating mode Efficiency, if an increase of the substituate flow still induces at least a rise of P > 65%,
wherein it is determined in the operating mode Economy, if an increase of the substituate flow still induces at least a rise of P > 85%, and

f) Output of the values determined in e) according to the range determined for the operating mode.

**Revendications**

1. Procédé pour la détermination d'un débit d'une solution de substitution, permettant d'obtenir une performance de purification d'un filtre à flux tangentiel d'une unité de traitement du sang pour des substances de masses moléculaires moyennes, comprenant les étapes suivantes :

a) sélection de la plage fixée au préalable sous la forme d'un mode opérationnel choisi parmi Optimum, Efficiency ou Economy ;
b) mesure d'un signal qui est produit par au moins une substance de masse moléculaire moyenne présente dans le flux du dialysat de l'unité de traitement du sang ;
c) variation du débit de la solution de substitution pendant la mesure selon b) ;
d) retransmission des débits de la solution de substitution et des valeurs de mesure correspondantes du signal de la au moins une substance de masse moléculaire moyenne dans le flux de dialysat à une unité centrale de calcul ;
e) corrélation des débits de la solution de substitution avec le signal de la au moins une substance de masse moléculaire moyenne, ladite corrélation comprenant :

la détermination de la performance de purification pour la au moins une substance de masse moléculaire moyenne (K0) avant variation du flux de substitution ;
la détermination de la performance de purification pour la au moins une substance de masse moléculaire moyenne (K1) après variation du flux de de substitution ;
la détermination de la variation du flux de de substitution en ml/min ($\Delta S$) ;
le calcul de la variation de la performance de purification (P) pour la au moins une substance de masse moléculaire moyenne en fonction de la variation du flux de substitution ($\Delta S$) selon :

$$\Delta K(0,1) = K1\text{-}K0$$

$$P = (\Delta K(0,1)/\Delta S)*100\%$$

où l'on détermine en mode opérationnel Optimum si une hausse du flux de substitution provoque au moins encore une hausse de 10 % < P < 40 %,
où l'on détermine en mode opérationnel Efficiency si une hausse du flux de substitution provoque au moins encore une hausse de 40 $\le$ P < 70 %,
où l'on détermine en mode opérationnel Economy si une hausse du flux de substitution provoque au moins encore une hausse de $P \geq 70\ \%$,

f) émission des valeurs déterminées au e) en fonction de la plage fixée pour le mode opérationnel.

2. Procédé selon la revendication 1, dans lequel ledit signal est un signal de mesure optique choisi dans le groupe constitué par une absorbance UV, une absorbance dans le domaine visible des longueurs d'ondes, un signal IR, un signal IR transformé en une mesure d'absorbance, une mesure de polarisation et un signal de fluorescence, ou un signal de mesure acoustique choisi dans le groupe constitué par des variations d'intensité acoustique ou de fréquence acoustique.

3. Procédé selon l'une des revendications précédentes, dans lequel un mode défini par l'utilisateur lui-même est choisi.

4. Procédé selon l'une des revendications précédentes, dans lequel le flux de la solution de substitution est modulé à des intervalles de temps prédéfinis ou librement sélectionnables.

5. Procédé selon la revendication 4, dans lequel les intervalles de temps prédéfinis restent identiques tout au long du traitement ou dans lequel les intervalles de temps prédéfinis augmentent jusqu'à la fin du traitement.

6. Procédé selon la revendication 5, dans lequel les intervalles de temps croissant jusqu'à la fin du traitement sont adaptés en fonction de la vitesse de variation du signal de mesure.

7. Procédé selon l'une des revendications précédentes, dans lequel l'évolution de la performance de purification est définie par la prise de différents points de mesure et une courbe est interpolée, les points de mesure étant pris de manière optimisée à fréquence constante ou par rapport à l'évolution escomptée.

8. Procédé selon l'une des revendications précédentes, dans lequel l'évolution de la performance de purification est comparée à une évolution modélisée enregistrée dans l'unité centrale de calcul et une mesure correctrice correspondante est proposée.

9. Procédé selon l'une des revendications précédentes, dans lequel les valeurs de mesure et les débits déterminés lors des séances de dialyse sont stockés pour chaque patient sur un support de données et sont accessibles pour une réutilisation.

10. Unité de traitement du sang, qui permet l'ajustement de la performance de purification du filtre à flux tangentiel pour les substances de masses moléculaires moyennes au moyen de la mesure en ligne d'un signal qui est en corrélation avec une purification de masse moléculaire moyenne, et qui comprend :

   - un filtre à flux tangentiel ;
   - un dispositif de mesure pour la mesure continue dudit signal d'au moins une substance de masse moléculaire moyenne présente dans le flux de dialysat de l'unité de traitement du sang, ledit dispositif de mesure pouvant mesurer l'absorbance UV, l'absorbance dans le domaine visible des longueurs d'ondes, un signal IR, un signal de fluorescence, l'intensité acoustique et/ou la fréquence acoustique ;
   - des moyens pour la variation du débit de la solution de substitution pendant la mesure continue ;
   - des moyens pour la retransmission des débits de la solution de substitution et des valeurs de mesure d'absorbance correspondantes de la au moins une substance de masse moléculaire moyenne dans le flux de dialysat à une unité centrale de calcul ;
   - une unité centrale de calcul ;
   - des moyens pour la corrélation des débits de la solution de substitution avec l'absorbance de la au moins une substance de masse moléculaire moyenne sur l'unité centrale de calcul, ladite corrélation comprenant :

      la détermination de la performance de purification pour la au moins une substance de masse moléculaire moyenne (K0) avant variation du flux de substitution ;
      la détermination de la performance de purification pour la au moins une substance de masse moléculaire moyenne (K1) après variation du flux de substitution ;
      la détermination de la variation du flux de substitution en ml/min ($\Delta S$) ;
      le calcul de la variation de la performance de purification (P) pour la au moins une substance de masse moléculaire moyenne en fonction de la variation du flux de substitution ($\Delta S$) selon :

$$\Delta K(0,1) = K1\text{-}K0$$

$$P = (\Delta K(0,1)/\Delta S)*100\ \%$$

où l'on détermine en mode opérationnel Optimum si une hausse du flux de substitution provoque au moins encore une hausse de 10 % < P < 40 %,
où l'on détermine en mode opérationnel Efficiency si une hausse du flux de substitution provoque au moins encore une hausse de 40 $\leq$ P < 70 %,
où l'on détermine en mode opérationnel Economy si une hausse du flux de substitution provoque au moins encore une hausse de P $\geq$ 70 %,

- et un dispositif d'affichage pour indiquer le résultat de la corrélation entre les débits de la solution de substitution et l'absorbance.

11. Unité de traitement du sang selon la revendication 10, comprenant en outre :

des moyens pour la régulation automatique du débit de la solution de substitution à l'aide du résultat déterminé lors de la corrélation.

12. Unité de traitement du sang selon la revendication 10 ou 11, comprenant en outre :

une possibilité de sélection grâce à laquelle l'utilisateur peut choisir un mode opérationnel dans le groupe constitué par le mode Economy, le mode Efficiency et le mode Optimum ou l'option de définir un mode opérationnel, la possibilité de sélection étant un commutateur à actionner par l'utilisateur ou un champ de saisie sur une surface utilisateur de l'affichage de l'unité centrale de calcul.

13. Procédé pour la détermination d'un débit d'une solution de substitution, permettant d'obtenir une performance de purification d'un filtre à flux tangentiel d'une unité de traitement du sang pour des substances de masses moléculaires moyennes, comprenant les étapes suivantes :

a) sélection de la plage fixée au préalable sous la forme d'un mode opérationnel choisi parmi Optimum, Efficiency ou Economy ;
b) mesure d'un signal qui est produit par au moins une substance de masse moléculaire moyenne dans le flux du dialysat de l'unité de traitement du sang ;
c) variation du débit de la solution de substitution pendant la mesure selon b) ;
d) retransmission des débits de la solution de substitution et des valeurs de mesure correspondantes du signal de la au moins une substance de masse moléculaire moyenne dans le flux de dialysat à une unité centrale de calcul ;
e) corrélation des débits de la solution de substitution avec le signal de la au moins une substance de masse moléculaire moyenne, ladite corrélation comprenant :

la détermination de la performance de purification pour la au moins une substance de masse moléculaire moyenne (K0) avant variation du flux de substitution ;
la détermination de la performance de purification pour la au moins une substance de masse moléculaire moyenne (K1) après une première variation du flux de substitution ;
la détermination de la performance de purification pour la au moins une substance de masse moléculaire moyenne (K2) après une deuxième variation du flux de substitution ;
le calcul de la variation de la performance de purification (P) pour la au moins une substance de masse moléculaire moyenne selon :

$$\Delta K(0,1) = K1\text{-}K0$$

$$\Delta K(1,2) = K2\text{-}K1$$

$$P = (\Delta K(1,2)/\Delta K(0,1))*100\%$$

où l'on détermine en mode opérationnel Optimum si une hausse du flux de substitution provoque au moins encore une hausse de P > 25 %,
où l'on détermine en mode opérationnel Efficiency si une hausse du flux de substitution provoque au moins encore une hausse de P > 65 %,
où l'on détermine en mode opérationnel Economy si une hausse du flux de substitution provoque au moins encore une hausse de P > 85 %,

f) émission des valeurs déterminées au e) en fonction de la plage fixée pour le mode opérationnel.

**Figur 1**

## Figur 2

## Figur 3

## Figur 4

```
┌─────────────────────────────┐      ┌─────────────────────────────┐
│      Blutfluss (QB)          │      │  Messsignal das Information  │
│     Hämatokrit (HCT)         │      │  über die Reinigungsleistung │
│    Total Protein (TP)        │      │          liefert             │
│  Filtereigenschaften (FE)    │      │                              │
└─────────────────────────────┘      └─────────────────────────────┘
               │
               ▼
┌─────────────────────────────┐
│      Maximal mögliche        │
│   Substitutionsrate (QSUB)   │
│    Ultrafiltrationsrate (QUF)│
│ Transmembrane Flussrate (QTM)│
└─────────────────────────────┘
               │
               ▼
┌─────────────────────────────────────┐
│   Variation von QSUB, QUF, QTM      │
└─────────────────────────────────────┘
               │
               ▼
┌─────────────────────────────┐      ┌─────────────────────────┐
│ K                            │      │      Funktion, die      │
│                              │      │  Reinigungsleistung     │
│                              │      │   und Flussrate in      │
│                          Q   │      │   Verbindung setzt.     │
└─────────────────────────────┘      └─────────────────────────┘

                              ┌─────────────────────────┐
                              │    Operationsmodus       │
                              └─────────────────────────┘
               │
               ▼
┌─────────────────────────────────────┐
│   Wahl des Optimalen Wertes für      │
│        QSUB, QUF, QTM                │
└─────────────────────────────────────┘
```

**Figur 5**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20080215247 A1
- WO 2006011009 A2
- EP 1175917 A1
- US 20050251086 A

**In der Beschreibung aufgeführte Nicht-Patentliteratur**

- **PORT et al.** *AJKD,* 2001, vol. 37, 276-86
- **LOCATELLI et al.** *Kidney Int.,* 1999, vol. 55, 286-93